(19) Europäisches Patentamt

European Patent Office

Office européen des brevets

(11) **EP 4 368 987 B1**

(12) **EUROPEAN PATENT SPECIFICATION**

(45) Date of publication and mention
of the grant of the patent:
**15.10.2025 Bulletin 2025/42**

(21) Application number: **22206920.5**

(22) Date of filing: **11.11.2022**

(51) International Patent Classification (IPC):
***G01N 33/00*** *(2006.01)*

(52) Cooperative Patent Classification (CPC):
**G01N 33/0034**

(54) **GAS SENSING DEVICE AND METHOD FOR SENSING A TARGET GAS**

GASERFASSUNGSVORRICHTUNG UND VERFAHREN ZUR ERFASSUNG EINES ZIELGASES

DISPOSITIF DE DÉTECTION DE GAZ ET PROCÉDÉ DE DÉTECTION D'UN GAZ CIBLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**15.05.2024 Bulletin 2024/20**

(73) Proprietor: **Infineon Technologies AG
85579 Neubiberg (DE)**

(72) Inventors:
• **SUKIANTO, Tobias
80636 München (DE)**
• **SCHOBER, Sebastian
89073 Ulm (DE)**
• **CARBONELLI, Cecilia
81477 München (DE)**
• **MITTERMAIER, Simon
85659 Forstern (DE)**

(74) Representative: **Hersina, Günter et al
Schoppe, Zimmermann, Stöckeler
Zinkler, Schenk & Partner mbB
Patentanwälte
Radlkoferstrasse 2
81373 München (DE)**

(56) References cited:
• PAN XIAOFANG ET AL: "A fast and robust
mixture gases identification and concentration
detection algorithm based on attention
mechanism equipped recurrent neural network
with double loss function", SENSORS AND
ACTUATORS B: CHEMICAL, ELSEVIER BV, NL,
vol. 342, 24 April 2021 (2021-04-24),
XP086585450, ISSN: 0925-4005, [retrieved on
20210424], DOI: 10.1016/J.SNB.2021.129982

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Examples of the present disclosure relate to a gas sensing devices for sensing a target gas in a gas mixture. Further examples related to methods for sensing a target gas in a gas mixture. Some examples relate to an attention-based architecture for robust predictions of a gas sensing device.

Gas sensing devices are used for detecting a specific gas in a gas mixture, e.g., in the environment of the sensing device, or for determining the concentration of a specific gas in the gas mixture. For example, chemiresistive multi-gas sensors arrays are an efficient and cost-savvy technology for measuring gases and evaluating air quality. For example, in gas sensor array, each sensor may be functionalized with different chemicals so that different sensitivities and a different behavior of the response can be observed depending on the gas interacting with the sensor surface. Signals, which are caused by chemical processes between the sensor materials and the air are measured and then translated into gas concentrations by special algorithms, often using artificial intelligence, to differentiate between gases. For example, Support Vector Machine (SVM) / Support Vector Regression (SVR), Random Forests and Linear/Logistic Regression, as well as more modern, but shallow Neural Network Techniques, such as Feed Forward Neural Networks, RNNs and light CNN approaches are used for evaluating sensing signals.

The article "PAN XIAOFANG ET AL: "A fast and robust mixture gases identification and concentration detection algorithm based on attention mechanism equipped recurrent neural network with double loss function", SENSORS AND ACTUATORS B: CHEMICAL, vol. 342, 24 April 2021 (2021-04-24), DOI: 10.1016/J.SNB.2021.129982" discloses a gas identification and concentration detection algorithm consisting of an attention mechanism equipped recurrent neural network with double loss function.

In general, an improved trade-off between a high accuracy, a high selectivity with respect to different gasses, a high robustness and reliability, and a resource-efficient implementation in terms of cost, computational power and memory is desirable for gas sensing.

Examples of the present disclosure rely on the idea to determine an estimation of the concentration of a target gas based on a sequence of samples of a measurement signal by using a neural network comprising an attention layer to weight respective contributions of the samples to the estimation. Relying on a sequence of samples for determining the estimation allows the neural network considering a temporal evolution of the measurement signal for the estimation. In particular, the usage of an attention layer for weighting the contributions of the samples allows an individual weighting of different temporal portions of the measurement signal, so that the neural network may exploit temporal dependencies, in particular long-term temporal dependencies, for estimating the concentration.

The present disclosure provides a gas sensing device in accordance with independent claim 1 for sensing a target gas in a gas mixture. The gas sensing device comprises a measurement module configured for obtaining a measurement signal, the measurement signal being responsive to a concentration of the target gas in the gas mixture. The gas sensing device further comprises a processing module configured for determining, for each of a sequence of samples of the measurement signal, a set of features, the features representing respective characteristics of the measurement signal. Further, the processing module is configured for using a neural network for determining an estimation of the concentration of the target gas based on the sets of features determined for the samples of the sequence. The neural network comprises an attention layer to weight respective contributions of the samples to the estimation.

Further the present disclosure provides a method in accordance with independent claim 15 for sensing a target gas in a gas mixture, the method comprising the following steps: obtaining a measurement signal, the measurement signal being responsive to the concentration of the target gas in the gas mixture; determining, for each of a sequence of samples of the measurement signal, a set of features, the features representing respective characteristics of the measurement signal; using a neural network for determining an estimation of the concentration of the target gas based on the sets of features determined for the samples of the sequence, wherein the neural network comprises an attention layer to weight respective contributions of the samples to the estimation.

Advantageous implementations are defined in the dependent claims.

Examples of the present disclosure are described in more detail below with respect to the figures, among which:

Fig. 1    illustrates an example of a gas sensing device and an example of a method for sensing a target gas according to examples,

Fig. 2    illustrates an example of an attention layer,

Fig. 3    illustrates a further example of the attention layer,

Fig. 4    illustrates an example of a multi-head attention layer,

Fig. 5    illustrates an example of the neural network,

Fig. 6      illustrates an example of an input layer,

Fig. 7      illustrates an example of a feed forward layer,

Fig. 8      illustrates an example of a positional encoding layer,

Fig. 9      illustrates an example of a flatten layer,

Fig. 10     illustrates examples of schemes for training and using the neural network,

Fig. 11     illustrates a comparison between an example of the disclosed architecture and a GRU,

Fig. 12     illustrates examples of attention maps,

Fig. 13     illustrates a comparison of predictions by an example of the disclosed architecture and by a RNN.

Examples of the present disclosure are now described in more detail with reference to the accompanying drawings, in which the same or similar elements or elements that have the same or similar functionality have the same reference signs assigned or are identified with the same name. In the following description, a plurality of details is set forth to provide a thorough explanation of examples of the disclosure. However, it will be apparent to one skilled in the art that other examples may be implemented without these specific details. In addition, features of the different examples described herein may be combined with each other, unless specifically noted otherwise.

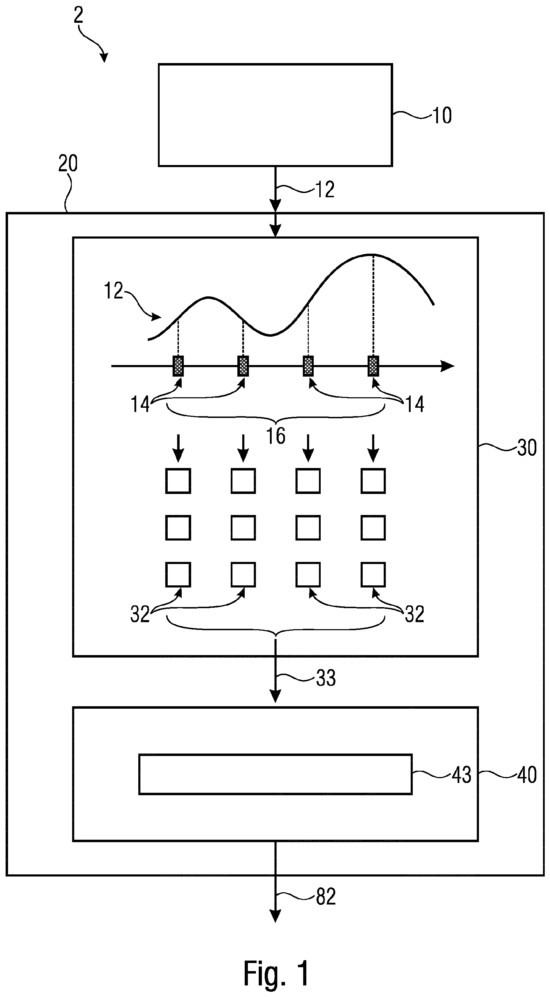

Fig. 1 illustrates an example of a gas sensing device 2 according to an example of the present disclosure. The gas sensing device 2 is for sensing one or more target gases in a gas mixture, e.g. in the environment of the gas sensing device 2. The gas sensing device 2 comprises a measurement module 10 configured for obtaining a measurement signal 12. The measurement signal is responsive to a concentration of the target gas in the gas mixture. The gas sensing device 2 comprises a processing module 20. The processing module 20 determines, in block 30, for each sample 14 of a sequence 16 of samples 14 of the measurement signal 12, a set 32 of features, the features representing respective characteristics of the measurement signal 12. The processing module 20 uses a neural network 40, e.g. an artificial neural network, for determining an estimation 82 of the concentration of the target gas based on the sets 32 of features determined for the samples of the sequence 16. The entirety of features of the sets 32 is referenced with sign 33 in Fig. 1. The neural network 40 comprises an attention layer 43 to weight respective contributions of the samples 14, e.g., contributions of the features determined for the samples 14 to the estimation 82.

It is noted that Fig. 1 may further serve as an illustration of a method for sensing a target gas in a gas mixture, in which the blocks of Fig. 1 represent steps of the method. Accordingly, Fig. 1 may serve as an illustration of a method comprising: a step 10 of obtaining a measurement signal 12, the measurement signal being responsive to the concentration of the target gas in the gas mixture; a step 30 of determining, for each of a sequence of samples of the measurement signal, a set of features, the features representing respective characteristics of the measurement signal; a step 40 of using a neural network for determining an estimation of the concentration of the target gas based on the sets of features determined for the samples of the sequence, wherein the neural network comprises an attention layer 43 to weight respective contributions of the samples to the estimation.

It is noted that the grouping of block/step 30 and block/step 40 into block 20/step 20 is optional and merely represents an illustrative example.

The features and functionalities described in the following may be optionally be part of or implemented by the gas sensing device 2 or the method for sensing a target gas.

For example, the neural network 40 is a trained neural network the parametrization or a model of which is obtained using training data.

For example, the measurement module 10 obtains the measurement signal 12 by receiving the measurement signal 12, e.g. from a sensing module, which is connected to the measurement module 10, or by determining the measurement signal 12 itself. For example, the measurement module 10 comprises a sensing module providing the measurement signal 12. The measurement signal 12 may be an analog or a digital signal. In the former case, the processing module 20 may sample the measurement signal 12 to obtain the sequence of samples 16.

For example, the measurement signal 12 may comprise, for each sample, one or more measurement values. For example, each of the measurement values may represent a resistance of a chemoresistive gas sensing unit.

For example, each sample 14 of the sequence 16 may be associated with a respective time instance, or time stamp. In other words, the sequence 16 may represent a temporal evolution of the measurement signal 12 over a time period covered by the sequence.

For example, the neural network 40 may process the sets 32 of features of the samples 14 sequence 16 in

3

parallel. In other words, the entirety of features of the sets 32 of features of the sequence 16 may provided to the neural network 40 as one set of input features to determine the estimation 82. Accordingly, the estimation 82 may rely on information about a period of the measurement signal 12 covered by the sequence 16. By processing the sets 32 in parallel, a fast processing is possible while exploiting the advantage of using a time series for determining the estimation 82.

For example, a dimension of a set of input features, e.g. a dimension of an input layer, of the neural network 40 may be the length of the sequence 16 times the number of features per set 32 of features.

For example, the measurement signal 12 may be affected not only by the true concentration of the target gas at the time of measuring the signal, but may further be affected by the previous evolution of the gas mixture, i.e. by the history. For example, the measurement signal 12 may be measured using one or more chemo-resistive gas sensing units. A chemo-resistive gas sensing unit may comprise a sensing layer, which is, during operation, exposed to the gas mixture, and which is sensitive to one or more target gases. For example, gas molecules of the target gas adsorbing at a surface of the sensing layer may result in a change of the electronic resistance of the sensing layer, which may be measured for obtaining the measurement signal 12. However, the sensing layers may degrade over time, e.g., due to oxidation. Furthermore, the molecules adsorbed at the sensing layers may influence the sensitivity to future changes of the concentration of the target gas. For example, a desorption of the adsorbed gas molecules may be comparatively slow relative to the adsorption, so that the sensing layer may saturate. Different types of gas sensing devices may be prone to similar or different effects. In any case, the sensitivity of a gas sensing device may be prone to reversible or irreversible degradation processes, so that the measurement signal 12 may depend on the history of the gas sensing device.

By determining the estimation 82 on the basis of the sequence 16 of samples of the measurement signal 12, the history, e.g., the previous exposure of a sensing unit to the target gas or another gas, may be taken into account by the neural network 40. In particular, by means of the attention layer 43, the sensitivity of the neural network may be focused onto specific portions of the measurement signal 12, i.e. onto specific sets of features, resulting in a more accurate estimation of the concentration. To this end, the neural network 40, and in particular the attention layer 43 as a part thereof, may be trained on a training data set. In other words, using the attention layer, the neural network may exploit the history of the measurement signal 12 to determine the estimation 82.

In other words, examples of the present disclosure employ an attention-based architecture for the estimation of gas concentrations. Compared to currently used Recurrent Networks, the disclosed architecture enables improved performance thanks to its capability to better exploit the history of the sensor signals. In particular, the attention based network structure, e.g. a multi-head structure as described below, may be exploited to judiciously learn in parallel portions of the sensor signals' history that are relevant to the final air quality prediction. The disclosed structure may keep both the overall processing latency and memory footprint of the parameters space at very low levels.

For example, the sequence 16 may be considered as a time-series defined as a sequence of observations that tracks a sample or a signal with respect to time. Prediction of time-series is achievable by creating predictive models that observe past values of the data to forecast future values. Examples of the present disclosure rely on the idea to solve such time series problem by using an attention layer, e.g. a regression encoder-based architecture, which may in examples be based on a multiple self-attention mechanism. A regression encoder is a supervised learning algorithm that enables sequential processing similar to recurrent neural Networks which however relies on self-attention instead of the recursive properties. A regression encoder has the benefit of parallelization and fast processing due to its non-recursive properties.

In other words, examples of the disclosed approach use a neural network based regression architecture to process the temporal dependent sensor output signals in order to predict the concentration of gases. In contrast to full transformer architectures known from Natural Language Processing [1], examples of the present disclosure may employ an encoder architecture for gas sensing. Examples of the disclosure rely on the finding that the encoder architecture can process an input sequence of variable length without exhibiting a recurrent structure. This allows our architecture to be highly parallelizable. For example, the decoder used in the field of Natural Language processing, mainly for translation, may be unnecessary, since it is used to produce sequential outputs with respect to sequential inputs. The encoder's task may be to transform each sensor signal from the input sequence and to form a context independent representation to a multiple context dependent representation. The key purpose of this mechanism is to extract information about how relevant a sensor signal, e.g. a sample of the signal, is to other sensor signals, e.g. another sample, in the sequence. This allows the algorithm to focus on different key parts of the sequence for prediction tasks and increase its ability to learn long-term dependencies.

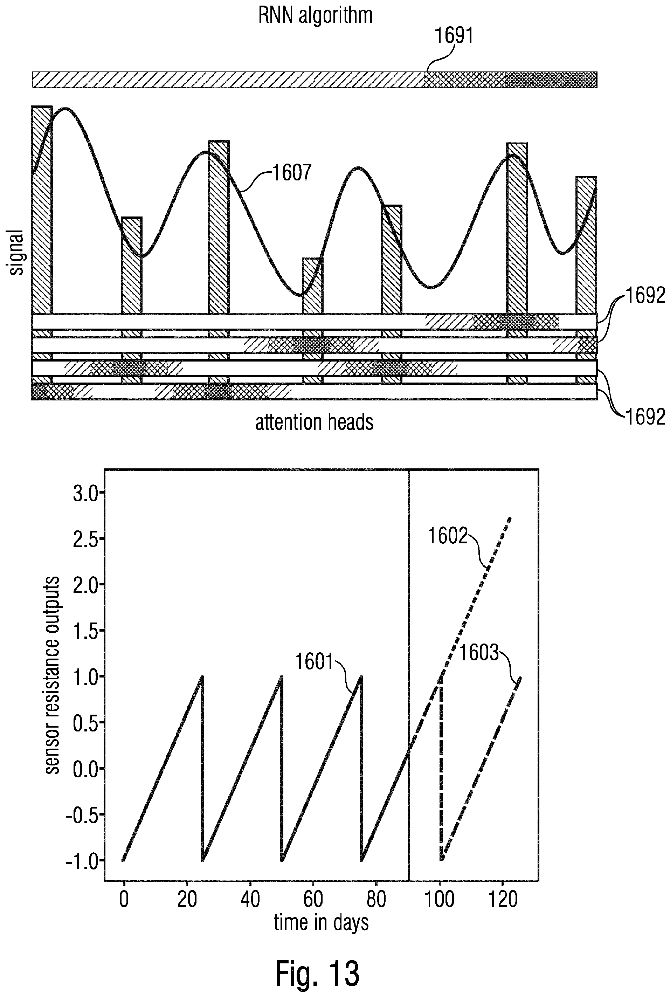

Fig. 13 illustrates the superior ability of examples of the disclosed attention-based architecture to predict sensor signals for timeseries over GRU based architectures. While the other architectures, such as the GRU, suffer from forgetting, the attention-based architecture is able to remember past seasonality and correctly predict the sensor signals. In Fig. 13, the upper panel illustrates an example of a sensor signal 1607 and, in greyscale coding, the contribution of individual parts of the sensor signal 1607 to the result of the estimation of the concentration. While the scale 1691 indicate the respective contributions for the case of an RNN network architecture, the bars 1692 illustrate the attention as determined by four individual attention heads, which will be described in more detail later. In other words, the upper panel illlustrates the Focus of the RNN and attention-based algorithms on the signal history. While the RNN, for example a GRU, tends to 'forget' earlier parts of the signal history over time, the attention-based architecture can focus on different parts of the signal simultaneously by using the attention layer, in particular various attention heads. The lower panel shows demonstrates how this focus on long-term dependencies of the attention-based architecture increases performance in the application area of time-series forecasting. Curve 1601 shows the ground-truth of a signal, curve 1602 a prediction based on GRU, and curve 1602 an example of a prediction using the herein disclosed attention-based architecture.

According to examples, the attention layer 43 is configured for using the sets 32 of features determined for the samples 14 of the sequence for determining weights for weighting the contribution of one of the samples to the estimation. For example, the attention layer 43 uses the features determined for all samples of the sequence 16 as an input for determining the weights.

In the following, examples for the implementation of the neural network 40 are described. The neural network may rely on a model, e.g. a trained model, which is trained to estimate the concentrations of the one or more target gases.

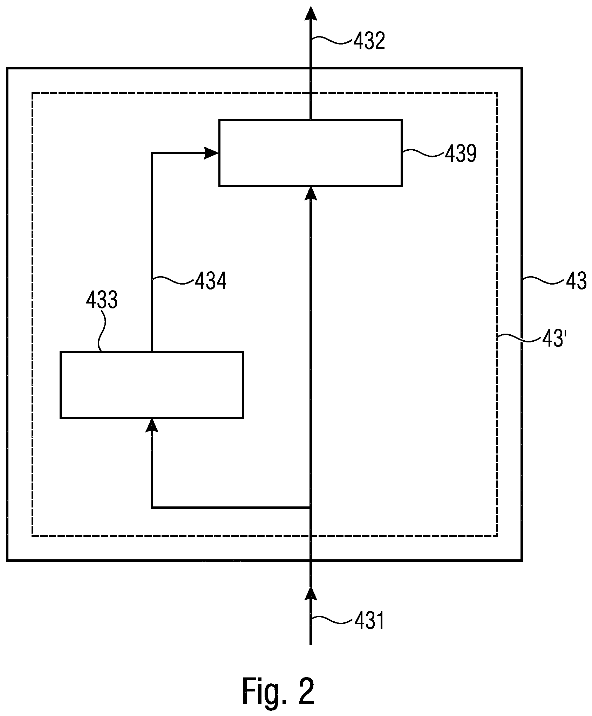

Fig. 2 shows a block diagram for an example of the attention layer 43. According to the example of Fig. 2, the attention layer 42 comprises a weight determination block 433 configured for determining weights 434 based on the sets 32 of features determined for the samples of the sequence 16, and further comprises a weighting block 439, which uses the weights 434 for weighting the contribution of one or more or each of the samples 14 of the sequence 16 to the estimation 82.

According to the invention, the attention layer 43 determines a plurality of output features 432 of the attention layer. Block 439 may determine the output features 432 by weighting respective contributions of the sets 32 of features to each of the output features 432.

The processing module 20 determines the estimation 82 based on the output features 432. For example, the neural network may determine the estimation 82 based on the output features 432.

Accordingly, the attention layer 43 is configured for determining weights 434 for weighting the contribution of one or more or all of the samples to the estimation 82 based on the sets 32 of features determined for the samples 14 of the sequence 16, for example, based on the features determined for all samples 14 of the sequence 16. For example the attention layer 43 may use the sets 32 of features determined for the samples 14 of the sequence 16, e.g., directly or by using a set of input features 431 of the attention layer 43 derived from the sets 32, for determining the weights 434.

As illustrated in Fig. 2, the attention layer 43 receives a plurality of input features 431. The input features 431 of the attention layer 43 may be derived from the sets 32 of features of the sequence 16 by one or more layers of the neural network 40, which precede the attention layer 32. The attention layer 43 determines the output features 432 based on the input feature 431. For example, weighting block 439 may determine the output features 432 by weighting respective contributions of the input features 431 to each of the output features 432.

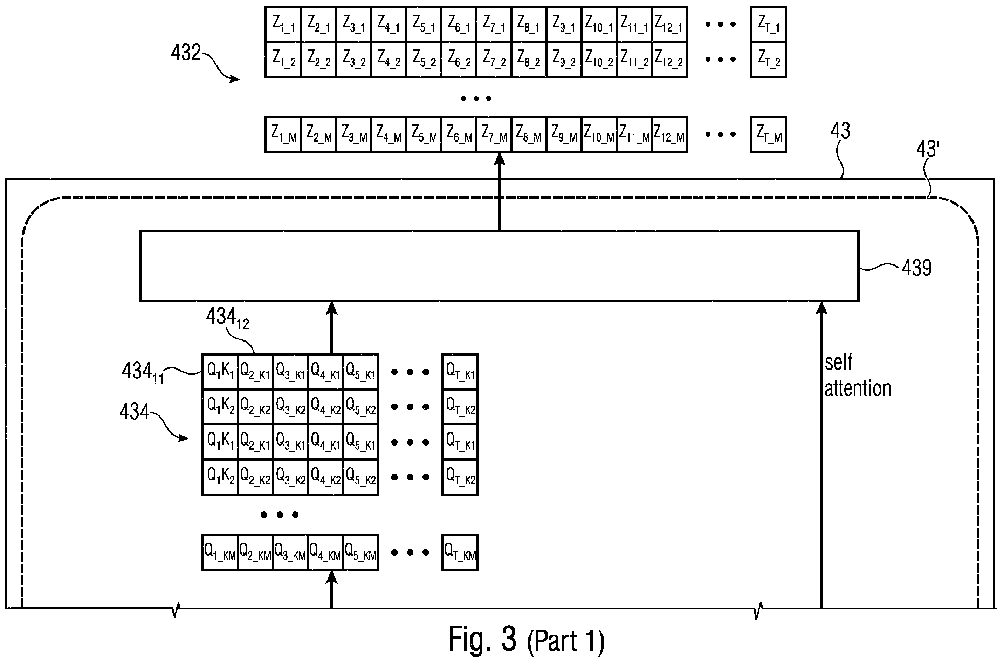

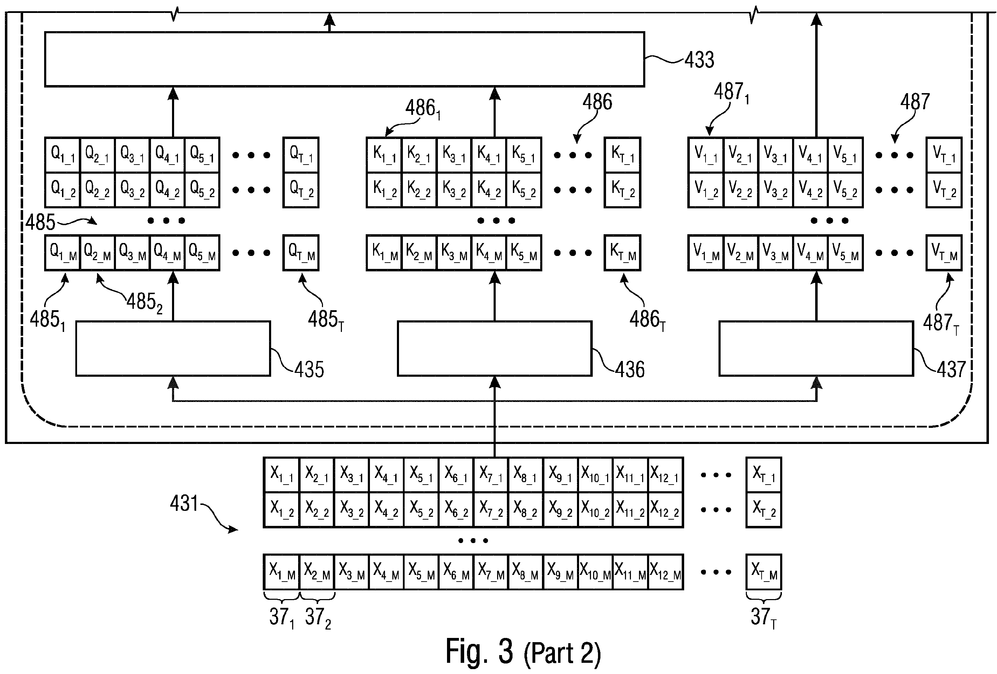

Fig. 3 illustrates further details of exemplary implementations of the attention layer 43 of Fig. 1 and Fig. 2. According to the example of Fig. 3, the plurality of input features 431 comprises a plurality of sets 37 of input features, e.g., one set of input features for each sample 14 of the sequence 16. In Fig. 3, the input features 431 comprise an exemplary number of T sets 37 of input features, the first, second and last sets being referenced in Fig. 3 using reference signs $37_1$, $37_2$, $37_T$ for illustrative purpose. According to the example of Fig. 3, weight determination block 433 is configured for determining, for each permutation, e.g., all permutations including repetitions, of two of the samples, a respective weight based on the sets 37 of input features associated with the two samples of the respective permutation. For example, weight determination block 433 determines, based on sets $37_1$ and $37_2$ a weight $434_{12}$, and based on set $37_1$, for a permutation comprising twice the set $37_1$, a weight $434_{11}$. The weights determined by weight determination block 433 may be part of a weight matrix, e.g. matrix $Q \times K^T$ as described below.

According to the invention and as illustrated in Fig. 3, the weighting block 439 determines the output features 432 of the attention layer by using the weights 434, i.e., the weights determined for each of the respective permutations, for weighting contributions of the input features 431 associated with the samples to the output features 432.

According to an example, the attention layer 43 of Fig. 3 may comprise block 435, e.g. referred to as query network, block 436, e.g. referred to as key network. Block 435 determines, for each of the samples, a first vector, e.g. referred to as query vector, by applying a first trained weight matrix, e.g. matrix $W_Q$ of the below described example, to the set 37 of input features associated with the respective sample. In Fig. 3, respective first vectors of a first sample and a last sample of the sequence are referenced using signs $485_1$ and $485_T$, respectively. Optionally, Block 435 may concatenate the first vectors to form a first matrix 485, e.g. matrix Q of equation 4 below. Block 436 determines, for each of the samples, a second vector, e.g. referred to as key vector, by applying a second trained weight matrix, e.g. matrix $W_K$, of the below described example, to the set 37 of input features associated with the respective sample. In Fig. 3, respective second vectors of a first sample and a last sample of the sequence are referenced using signs $486_1$ and $486_T$, respectively. Optionally, Block 436 may concatenate the first vectors to form a second matrix 486, e.g., matrix K of equation 5 below. The attention layer 43 determines the respective weight for the respective permutation of two of the samples by forming a product of the first vector associated with one of the two samples and the second vector associated with the other one of the two samples. For example, in Fig. 3, weight $434_{12}$ may be determined based on the first vector $485_2$ determined for the second sample of the sequence, and based on the second vector $486_1$ determined for the first sample of the sequence.

According to an example, in which the first vectors are concatenated to form a first matrix 485, and the second vectors are concatenated to form a second matrix 486, block 433 is configured for determining the weights 434 in form of a weight matrix by multiplying the first matrix 485 with a transposed version of the second matrix 486.

According to an example, block 433 normalizes the weights 434. For example, the normalization is performed with respect to the entirety of the weights 434, e.g. with respect to a range thereof. Additionally or altnernatively, block 433 applies a softmax function to the weights, e.g., the normalized weights. After normalizing and/or subjecting the weights to the softmax function, the weights may be used for weighting the contributions in block 439.

According to an example, the attention layer 43 of Fig. 3 may comprise block 437, e.g. referred to as value network. Block 437 is configured for determining, for each of the samples, a third vector, e.g. referred to as value vector, by applying a third trained weight matrix, e.g. matrix $W_V$ of the below described example, to the set 37 of input features associated with the respective sample. The result may correspond to matrix V of equation 6 below. In Fig. 3, respective second vectors of a first sample and a last sample of the sequence are referenced using signs $486_1$ and $486_T$, respectively. According to this example, block 439 determines the output features 432 of the attention layer 43 by using the weights 434 determined for the permutations for weighting the third vectors, e.g., for weighting respective components of the third vectors.

According to an example, the weights 434 determined for the permutations form a weight matrix, and the attention layer 43 is configured for concatenating the third vectors $487_{1,...,T}$ associated with the samples to form a value matrix 487. Further, according to this example, block 439 determines the output features 432 by multiplying the weight matrix 434 and the value matrix 487, e.g. using a matrix multiplication.

The functionality of attention layer 43 described up to this point with respect to Fig. 2 and Fig. 3 may, in examples, be referred to as self-attention. In other words, as indicated in Fig. 2 and Fig. 3, attention layer 43 may comprise a self-attention block 43', which determines output features 432 based on the input features 431 as described with respect to Fig. 2 and Fig. 3.

In other words, in examples, each of the self-attention blocks 43' may be implemented as a sequence to sequence operation where the input sequence, e.g. in form of input features 431, is transformed to an output sequence 432', e.g., with same length and dimensionality. Self-attention takes a weighted average over all input vectors. As described before, each element of the input sequence may be transformed, e.g., with linear transformation, to a value, key and query vector respectively. To obtain this linear transformation, three learnable weight matrices may be employed, e.g., each one with the same dimensions. These three matrices are referred to as $W_V$, $W_K$, and $W_Q$, respectively, e.g., three trained or learnable weight matrices that are applied to the same encoded input. The matrices $W_V$, $W_K$, and $W_Q$ may correspond to the third, second, and first trained weight matrices described before, respectively.

The second step in the self-attention operation may be to calculate a score by taking the dot product of every query vector and key vector in every permutation. The score determines how much focus to place on other parts of the sequence as we encode an element of the sequence at a certain position. As a third step, the scores may be normalized, e.g. by dividing them by a predetermined value, e.g. by 8, and then normalizing the results by passing them through a softmax operation. Softmax normalizes the scores by ensuring that the scores are positive and add up to 1. To filter out the irrelevant elements in a sequence when observing one element, we each value vector may be multiplied by the softmax score. The fourth step is to sum up the weighted value vectors, which calculates the output of the self-attention layer at this position.

Accordingly, the operation of the self-attention may, in examples, be summarized as follows: In a first step, each element of the input sequence is given 3 attributes: Value, Key, Query. In a second step, an element wants to find specific values by matching its queries with the keys. In a third step, the final value of query element is computed by averaging the accessed values. In a fourth step, the final value is transformed to have a new representation for query element.

For example, each input sequence element $x_1, ..., x_T \in R^M$ is transformed with learnable linear transformation into three different vector representations key $k1, ..., k_T \in R^{dH}$ query $q_1, ..., q_T \in R^{dH}$ and value $v_1, ..., v_T \in R^{dH}$. For example, each input sequence element $x_i$ corresponds to one set 37 of the sets of input features of the attention layer 43, T being, e.g., the length of the sequence 16 of samples, and M the number of features per set 37 of features. To obtain this linear transformation, three learnable weight matrices $W_Q$, $W_K$ and $W_V$ may be used, e.g., each with the same dimensions:

$$q_i = W_Q x_i \text{ ,with } W_Q \in R^{d_H \times M} \qquad (1)$$

$$k_i = W_K x_i, \text{ ,with } W_K \in R^{d_H \times M} \qquad (2)$$

$$v_i = W_V x_i, \text{ ,with } W_V \in R^{d_H \times M} \qquad (3)$$

$d_H$ corresponds to the dimension of the transformed vector representation. The resulting query, value and key vectors may then beconcatenated into Query matrix $Q$, Key matrix $K$ and Value Matrix $V$ , e.g., in following fashion:

$$Q = [q_1, ..., q_T]^T \text{ ,with } Q \in R^{T \times d_H} \qquad (4)$$

$$K = [k_1, ..., k_T]^T \text{ ,with } K \in R^{T \times d_H} \qquad (5)$$

$$V = [v_1, ..., v_T]^T \text{ ,with } V \in R^{T \times d_H} \qquad (6)$$

A score by multiplying $Q$ and $K$ is then calculated. The score determines how much focus on placing on other parts of the sequence should be given relative to an encoded element of the sequence at a particular position. The output of self attention, e.g., output features 432, may then produced, e.g. by weighting block 439, as follows:

$$\text{Attention}(Q, K, V) = \text{softmax}(\frac{Q \times K^T}{\sqrt{d_H}}) \times V \qquad (7)$$

The denominator of equation 7 is a normalization term that may reduce the scores for gradient stability. Softmax normalizes the scores for probability distribution by ensuring that the scores are positive and add up to one. For example, the weights 434 described above may be the result of the softmax function of equation 7. When observing one element, the value matrix is multiplied by the resulting probability matrix to filter out the irrelevant elements in a sequence.

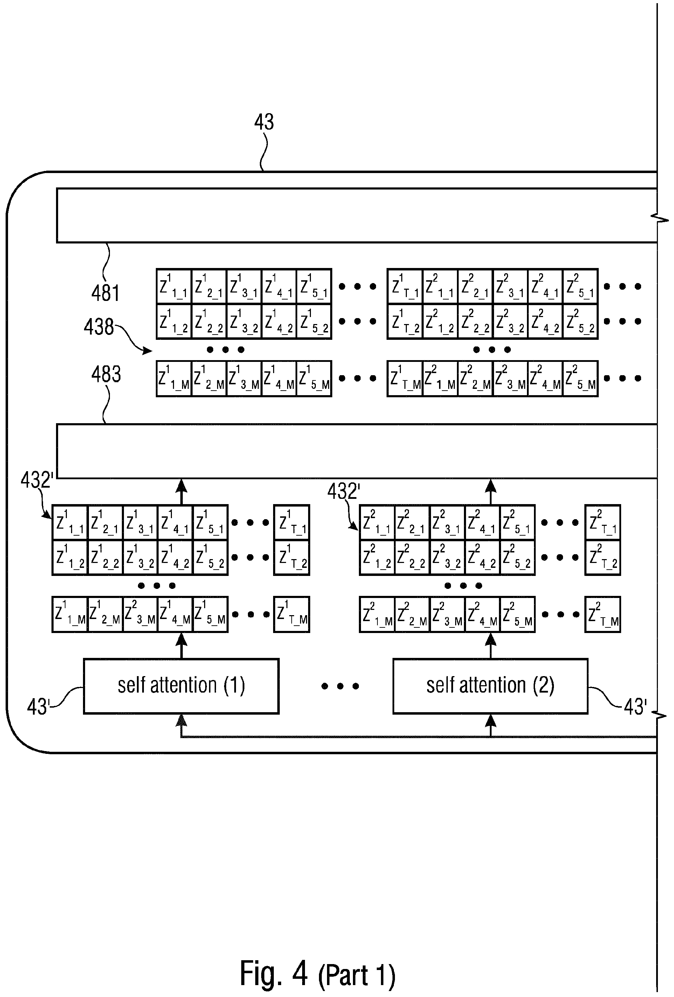

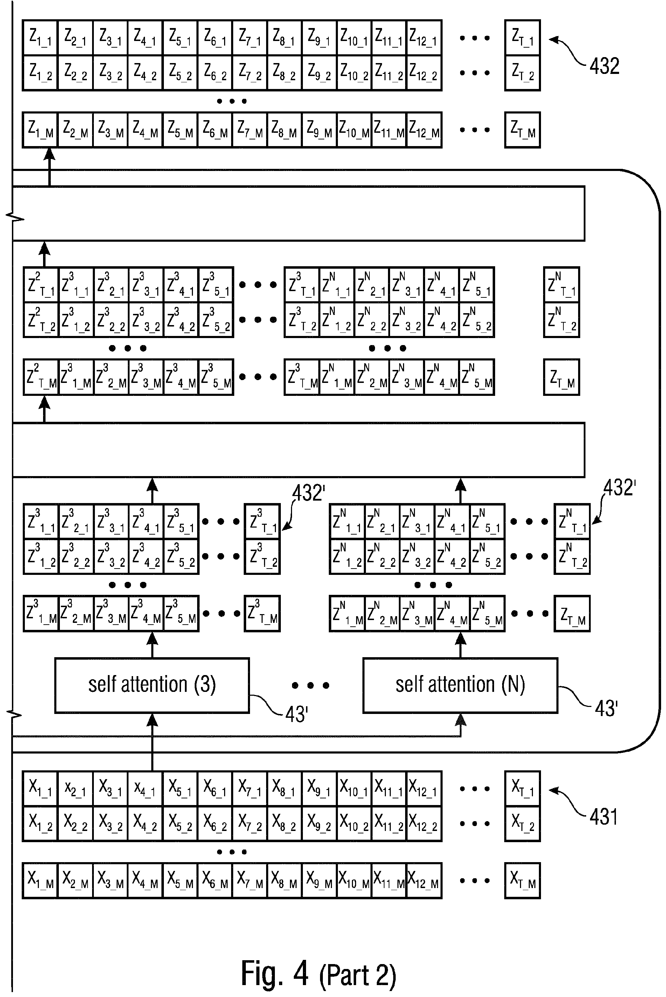

Fig. 4 illustrates another example of the attention layer 43, according to which the attention layer 43 comprises a plurality of self-attention blocks 43', wherein each of the self-attention blocks 43' is configured for determining a respective plurality of output features 432' based on the input features 431 of the attention layer, and wherein the attention layer 43 is configured for determining the plurality of output features 432 of the attention layer by using a fourth trained weight matrix 438 for weighting contributions of the output features 432' of the self-attention blocks 43' to the output features 432 of the attention layer 43.

For example, each of the self-attention blocks 43' is implemented as described with respect to Fig. 2 and/or Fig. 3. For example, the first, second, and third trained weight matrixes, which may be used by the self-attention blocks 43', may be individually trained for each of the self-attention blocks 43'. In other words, each of the self-attention blocks 43' may use respective first, second, and/or third trained weight matrixes.

For example, the attention layer 43 may concatenate, e.g. in block 483 of Fig. 4, the output features 432', which may each form a respective matrix, of the self-attention blocks 43' and multiply the fourth trained weight matrix 438 with the concatenated weights to obtain the output features 432.

In other words, in examples, the attention layer 43 may be implemented as a multi-head attention layer.

For example, a multi-head attention layer performs self-attention several times in parallel; each time with a different set of values, keys and queries. This processing expands the model's ability to focus on different positions and gives the architecture different representation subspaces. If self-attention is executed N times in parallel, then the output may be in the dimension [N, Number T of time steps of the sequence 16, Number of features]. In order to reduce the dimension back to [T, feature_dimensions] a linear transformation may applied by a learnable weight matrix, e.g. a positional feedforward layer with no activation function.

For example, the Multi-Head Attention (MHA) layer 43 performs self-attention e.g. block 43', $H$ times in parallel (and therefore may, e.g., also be referred to as multi-head self-attention layer, MHSA layer), each time with a different set of value matrix $V^1, ..., V^H$, key matrix $K^1, ..., K^H$, and query matrix $Q^1, ..., Q^H$ representation. This may expand the model's ability to focus on different positions and gives the architecture different representation subspaces. For example, each group of $\{V^i, K^i, Q^i\}$ performs the self-attention process. The output of MHSA may then be produced by projecting the concatenation of the outputs of the $H$ self-attention layers with a learnable weight matrix $W \in R^{(d \cdot H) \times M}$, which may correspond to the fourth trained weight matrix mentioned above, to the original input sequence's $X = [x_1, ..., x_T]^T \in R^{T \times M}$ dimension:

$$\text{MHSA}(\{Q_i, K_i, V_i\}_{i=1}^H) = [\text{Attention}(Q_1, K_1, V_1), ..., \text{Attention}(Q_H, K_H, V_H)]W \qquad (8)$$

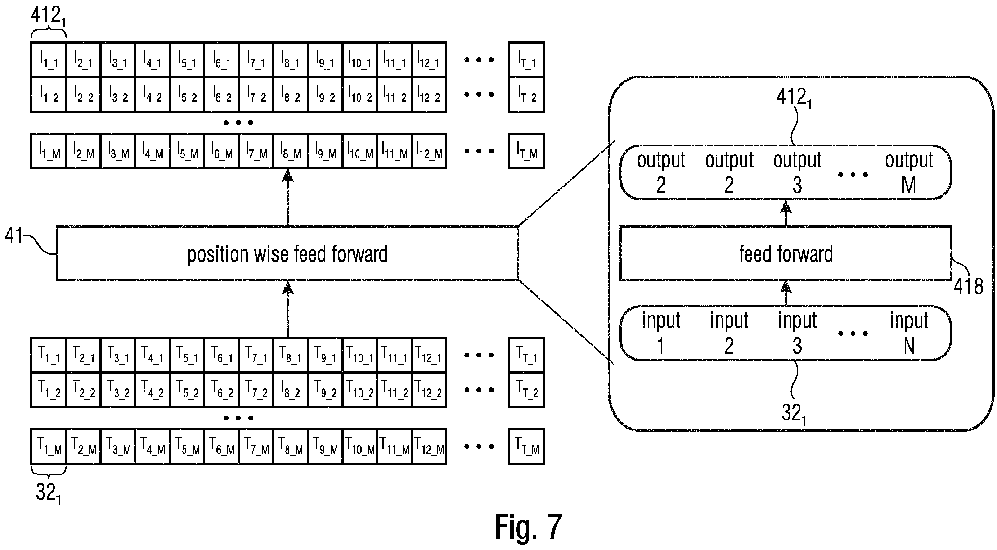

Optionally, the result of multiplying the fourth trained weight matrix 438 with the concatenated weights may be subjected to a feed forward layer 481 to obtain the output features 432, as illustrated in Fig. 4. For example, the feed forward layer 481 may be implemented as a positional feed forward layer, e.g., as described with respect to Fig. 7.

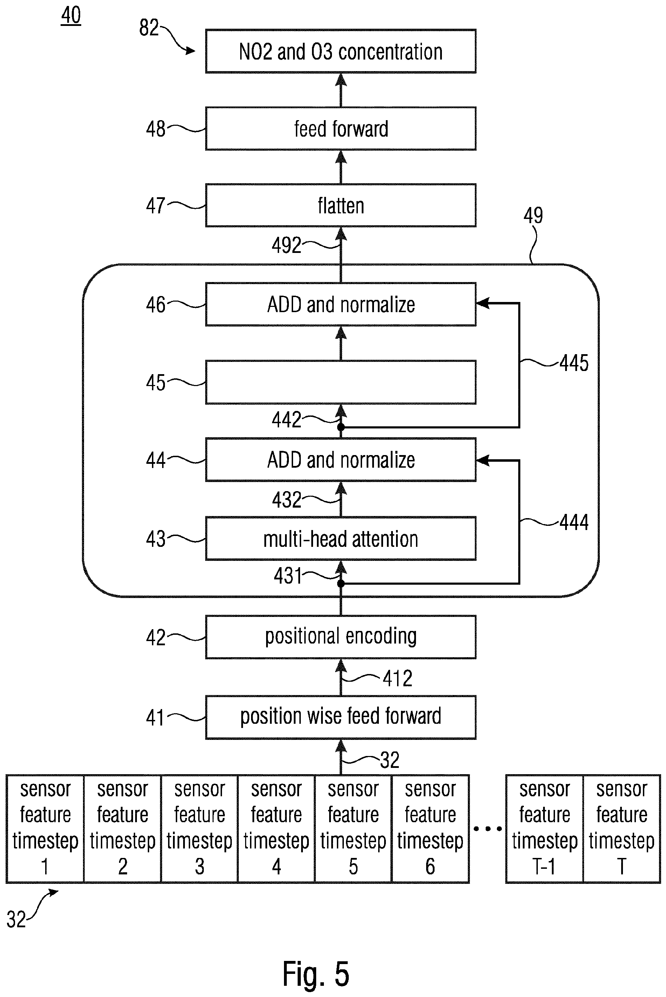

Fig. 5 illustrates an example of the neural network 40. According to the example of Fig. 5, the neural network may combine, in block 44 of Fig. 5, the output features 432 of the attention layer 43 with the input features 431 of the attention layer to obtain a plurality of combined features 442. For example, block 44 may combine the output features 432 and the input feature 431 element-wise with respect to respective matrixes, in which the input features and the output features are arranged. Optionally, block 44 may normalize the combined features, and provide the combined features 442 as normalized combined features.

Throughout this description, an element-wise operation may refer to an operation on elements of corresponding, or collocated, positions in respective matrixes. E.g., the operation may be performed individually on each position of a set of equally-sized matrixes, receiving, as an input, each one element of two or more matrixes, which elements have equivalent positions within the two or more matrixes.

In the sense of combining the input features 431 and the output features 432, the output features 432 may be regarded as a residual of the input features. Accordingly, the combining of the input features 431 and the output features 432 may be considered as a residual connection 444.

According to an example, the neural network 40 further comprises a feed forward layer 45, which may be arranged downstream of the attention layer, i.e. the input features of which are based on the output features of the attention layer 43. For example the feed forward layer 45 may use the combined features 442 as input features. For example, the feed forward layer is a position wise feed forward layer.

According to an examples, the neural network comprises a residual connection 445 between the input and the output of the feed forward layer 45. The residual connection 445 may be implemented similarly as residual connection 444. For example, the neural network may combine, in block 46 of Fig. 5, the input features and the output features of layer 45, e.g., by adding them element-wise. Optionally, block 46 may normalize the combined features.

The residual connections 444, 445 may provide for a better gradient in training the neural network.

The residual connections 444, 445 are optional, and may be implemented individually from each other. Further, the feed forward layer 45 is optional, and may be implemented independent from the implementation of the residual connections 444 and/or 445.

As illustrated in Fig. 5, the attention layer 43, the optional residual connection 444, 445 including blocks 44 and 45, and the optional feed forward layer 46 may form a block referred to as attention coding block 49, which provides output features 492. In examples, the attention coding block 49 may be operated in a loop-wise manner by repeating the operation of the attention coding block 49 one or more times. In other words, the output features 492 may be fed to the attention coding block as input features to determine an iterated version of the output features 492.

In examples, the neural network 40 may comprise, downstream to the attention layer and, if present, the optional layers 44 to 46, a feed forward layer 48. For example, the feed forward layer 48 may perform a classification on its input features to provide the estimation 82 on the concentration of the one or more target gases, e.g., $NO_2$ and/or $O_3$.

Optionally, a flattening layer 47 may be implemented preceding the feed forward layer 48, e.g. directly preceding the feed forward layer 48, or between the attention coding block 49 and the feed forward layer 48.

According to an example, the neural network further comprises a positional encoding layer 42 configured for determining, for each of the samples 14, a set of positional coded features based on the set 32 of features associated with the respective sample by coding, into the set of positional coded features, information about a position of the respective sample within the sequence 16 of samples. According to this example, the neural network 40 uses the positional coded features for, or as, the input features 431 of the attention layer 43.

For example, since the sequential data is inserted in the attention-based encoder coder 49 simultaneously, it is an option to teach the attention-based encoder coder 49 of the positional order of the sequence. Without the positional encoding layer, the attention-based model treats each sensor signal in the sequence as positionally independent from the other. Therefore, injecting positional information to the model may explicitly retain the information regarding the order of elements in the sequence. Positional encoding may maintain the knowledge of order of objects in the sequence.

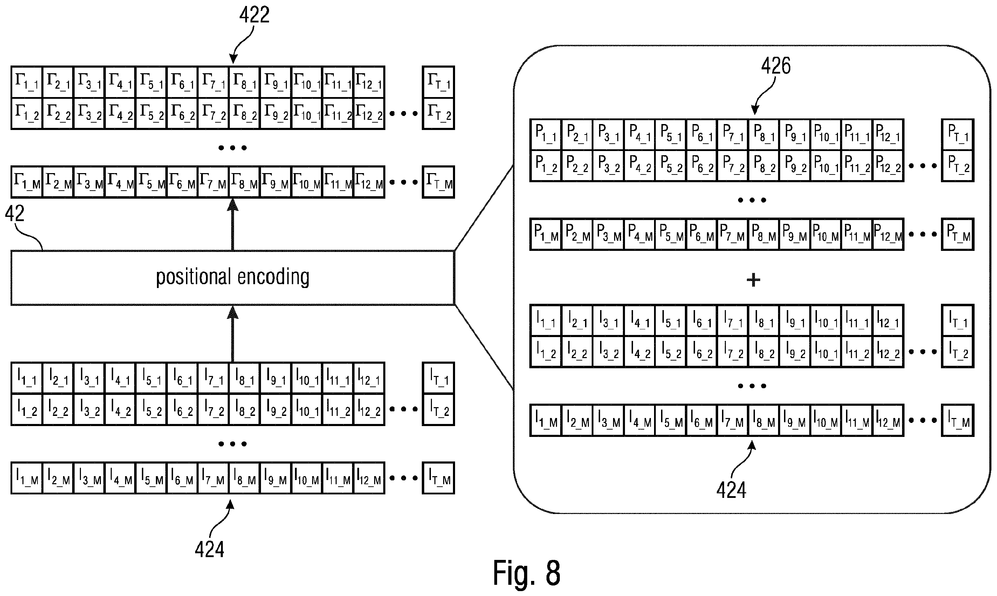

For example, one possibility to implement positional encoding in the input sequence, is to increase the feature dimension by one, where a single number such as the index value is used to represent an item's position. This however is restricted to shorter sequences and lower dimensional input features, because this approach would increase the number of parameters significantly and the index values would grow large in magnitude. Another implementation is described with respect to Fig 8.

According to an example, the neural network further comprises a feed forward layer 41 configured for applying a feed forward transformation to each of the sets 32 of features associated with the samples. In this example, the input features 431 of the attention layer are based on output features 412 of the feed forward layer 41. For example, the feed forward layer 41 applies the feed forward transformation individually to each of the sets 32 of features, e.g., the same feed forward transformation to each of the sets 32 of features. In other words, the feed forward layer 41 may be referred to as position-wise feed forward layer.

The positional encoding layer 42 may use the output features 412 to provide the input features 431 of the attention layer.

It is noted, that each of the feed forward layers 41, 45, 48 is optional, and may be implemented or combined with the further described features independent of other design choices.

In other words, as shown in Fig. 5, the architecture of the neural network 40 may comprise, or consist of, eight hidden layers. The input layer, i.e. the input features 32, may consist of a two-dimensional sensor output signal, in which the first dimension is temporal and the second dimension is the feature dimension. In the first dimension, temporal information is contained about the output signals of the sensor from the past.

The second layer 41 is optional and its depth may be subjected to the complexity of the input features.

The third layer 42 may be a positional encoding layer, which injects the model with knowledge of the positional ordering of the inputs.

The fourth layer 43 may be an attention layer, in particular a multi-head attention layer, which performs multiple self-attention on the inputs.

The fifth layer 44 may be a residual layer followed by a layer normalization from the outputs of the second layer and the third layer.

The sixth layer 45 may consist of a positional feed forward layer, which is optional.

The seventh layer 46 may be a residual layer followed by a layer normalization for the outputs of the sixth layer 45 and fifth layer 44.

The corpus of layers 43 to 46 may be defined as an encoder and can optionally be repeated, e.g., a predetermined number of times, and stacked on top of each other.

Afterwards, the features are flattened by flattening layer 47 by transforming the two-dimensional output of the Transformer Encoder to a one-dimensional vector. This layer can be optionally replaced with an average or max pooling layer, which takes the average of the two-dimensional tensor across the time dimension. The transformation axis is optional.

In other words, the flatten layer that is used to transform the features from a 2D matrix to a 1D vector can be replaced by a pooling layer.

Afterwards, a feed forward network, e.g., a dense layer, is applied, which may have the dimensionality of the number of the gases to be estimated.

According to examples, the positional encoding layer 42 is left out. This might slightly sacrifice performance or requires longer training time for the algorithm to converge, but may reduce the computational requirements. In other words, the positional encoding layer 42 is optional.

Furthermore, it is also possible to add and remove the positional feed forward layers in the architecture.

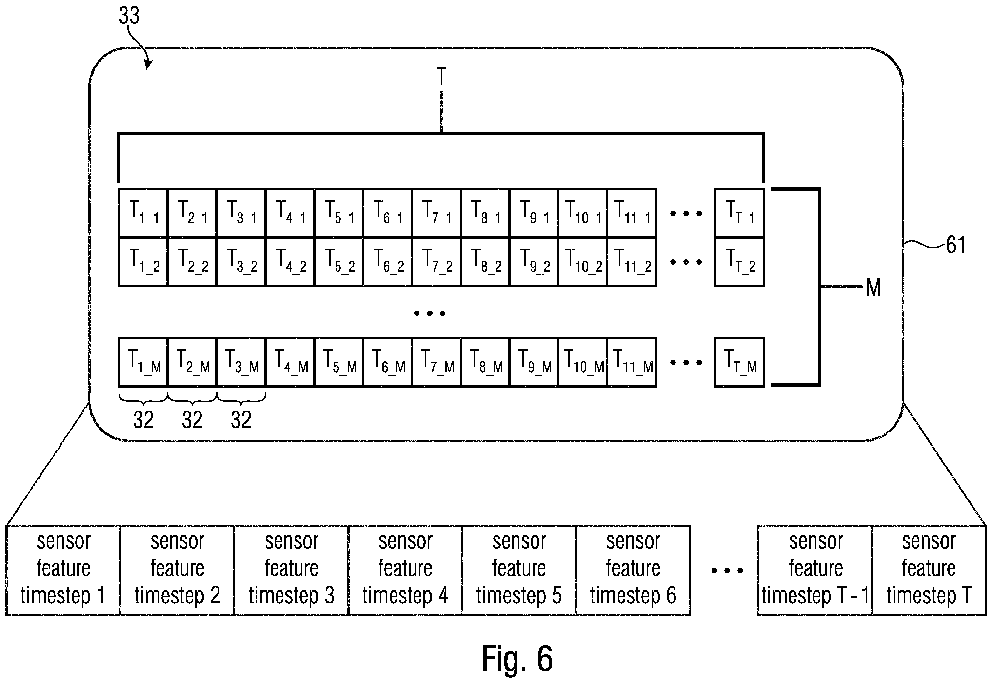

Fig. 6 illustrates an example of an input layer 61 of the neural network 40. For example, the sets 32 of features determined for the samples of the sequence 16 may be provided to the neural network 40 as input layer. In the example of Fig. 6, the length of the sequence 16, i.e. the number of samples of the sequence or the number of timesteps, is denoted as T, and the number of features per set 32, which may be referred to as sensor feature dimension, is M.

In other words, an input layer of the neural network may be configured to takes a time-series input with the feature dimension equal to the number of buffered sensor signals and outputs of the sensor. The length of the time-series input is a hyperparameter that may be chosen dependent on the problem statement, e.g. an application scenario of the gas sensing device. A longer input sequence usually enables more accurate estimations, however, is restricted by the available memory size.

Fig. 7 illustrates an exemplary implementation of the feed forward layer 41, implemented as positional feed forward layer. In the positional, or position-wise, feed forward layer 41 of Fig. 7, a feed forward layer 418, e.g., a dense layer, may be applied individually to each of the sets 32 of input features, as illustrated for the first set $32_1$ in Fig. 7, to obtain a respective set of output features, e.g. set $412_1$ of output features for the first set $32_1$. For example, a positional feed forward layer is a type of feed forward layer that enables the processing of two-dimensional input.

For example, the feed forward layer may increase, decrease, or maintain the feature dimension, i.e. the number of features of the individual sets of output features with respect to the number of features of the respective sets 32 of input features.

In other words, the feed forward layer 41 may comprise, or consist of, one dense layer 418 that applies to the feature dimension and leaves the time dimension untouched, which means the same dense layers may be used for each position item, e.g., each set 32 of features, in the sequence 16, so called position-wise.

For example, the feed forward layer may be a position-wise transformation that comprises, or consists of, a linear transformation and ReLU activation function. The activation function is optional and can be left out or replaced with other activation functions, such as GeLU, tanh, etc. Furthermore, we can also stack several positional feed forward layers on top of each other.

For example, the feed forward layer 418 may apply a trained weight matrix to each of the sets 32 of features.

Although the above description explains the feed forward layer with respect to layer 41, same description may optionally apply to the positional feed forward layer 45, and/or the feed forward layer 48, the input features in these cases being the output features of the respective preceding layer of the neural neural network. For example, each column of a matrix of input features may be regarded as one set of features in the above description.

Fig. 8 illustrates an exemplary implementation of the positional encoding layer 42. According to the example of Fig. 8, the sets of input features of layer 42, e.g., the sets 32 of feautes, or the output features 412 of the feed forward layer 41, which may equivalently comprise a respective set of features for each of the samples 14, may be concatenated to form a matrix 424, which matrix may be added in a position-wise manner with a position coding matrix 426 to obtain output features 422 of the positional encoding layer 42. For example the position coding matrix 426 may be a trained coefficient matrix.

In other words, a possible positional encoding scheme is to map each position, e.g. each set of input features, to a vector. Hence, the output of the positional encoding layer may be a matrix, where each column of the matrix represents an encoded position of the sequence. For each element in the sequence, the positional embedding layer may add each component of the sequence with a positional matrix. Positional embeddings can be either trained with the neural network, or they can be fixed and pre-computed. The benefits of pre-computed positional embeddings is the need of less trainable parameters.

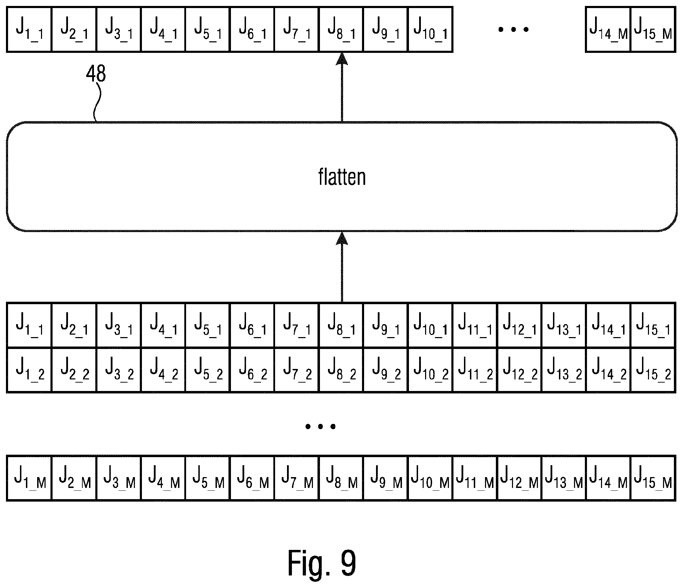

Fig. 9 illustrates an exemplary implementation of the flattening layer 48. The flatten layer is 48 flattens its input features to provide output features in a one-dimensional structure. In other words, the flatten layer may be used to make the two-dimensional input one-dimensional.

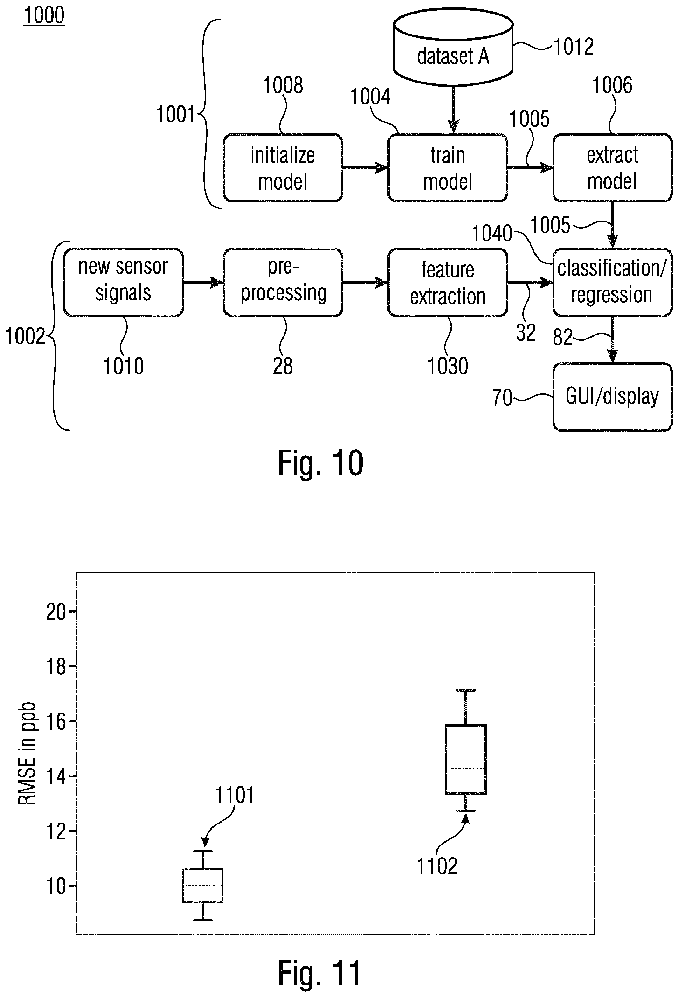

Fig. 10 illustrates a flow chart of a scheme 1000 for training and employing a model 1005 for the neural network according to an example. The training phase is referenced using sign 1001. In step 1008 of scheme 1000, a model is initiated. Subsequently, the model is trained in step 1004 using a data set 1012. In step 1006, the trained model 1005 is extracted. For example the extracted model may be stored on the gas sensing device 2. The training 1004 may, e.g., be performed independent of the gas sensing device 2.

In an operation phase 1002, as it may be performed by gas sensing device 2, sensor signals are obtained in step 1010, e.g. performed by block 10 as described with respect to Fig. 1. The sensor signals may optionally be preprocessed in step 28, e.g. by performing a calibration or compensation, e.g., an offset correction. Step 28 may optionally be part of step 1010 or step 1030, and may accordingly be performed by block 10, 20, or 30 of Fig. 1. Subsequently, features 32 are extracted in step 1030, which may be performed by block 30 of Fig. 1. In step 1040, the estimation 82 is determined using the neural network 40, which, to this end, employs the model 1005. Optionally, the result may be output to an interface in step 70, e.g. a GUI or a display.

For example, in the training phase illustrated in Fig. 10, measurement database, regression labels and information from the sensors are given to CPU or GPU to optimize parameters of the model architecture of the neural network, e.g. the architecture of Fig 5. The trained architecture 1005 is then put in the deployment algorithm, as shown in Fig. 10, to predict air quality based on signals from multi-gas and some other sensors in real time.

For example, with a multi-gas sensor array and the algorithm embedded in a portable device, for example in a smartphone, the user can read the air quality level with the lowest latency on the go.

The operation scheme 1002 and the training scheme 1001 may be implemented and performed individually, i.e., independent from each other.

In an example, a transfer learning mechanism can be implemented. In the transfer learning process, a limited set of additional data points that are acquired during the run time of a sensor can be used to update the attention-based encoder 1005 model on-the-fly.

Fig. 11 illustrates a comparison of the reliability in estimating the concentration of a target gas between an example of the neural network 40 as disclosed herein, in particular the architecture shown in Fig. 5, and a neural network relying on a gated recurrent uint (GRU). The diagram of Fig. 10 shows the root mean square error (RMSE) in ppb of the neural network 40, referenced using sign 1101, and of the GRU, reference sign 1102. The testing dataset are from sensors with different physical structure. The lower RMSE 1101 indicates that the neural network 40 is more robust to distribution shifts than the GRU and suggests more stable behavior towards sensor ageing and sensor-to-sensor variations.

Furthermore, a Transfer Learning embodiment was tested and showed a higher adaptability of the neural network 40 to such an implementation than for the GRU case with more stable results.

Overall, the neural network 40 according to the present disclosure may provide a high stability and data efficiency of environmental sensing algorithms.

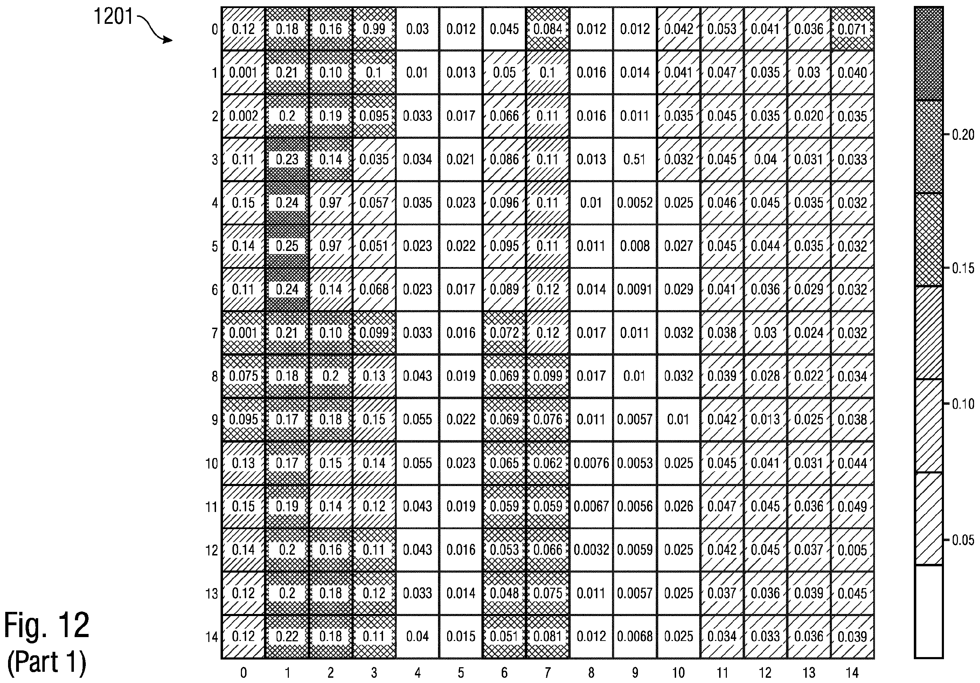

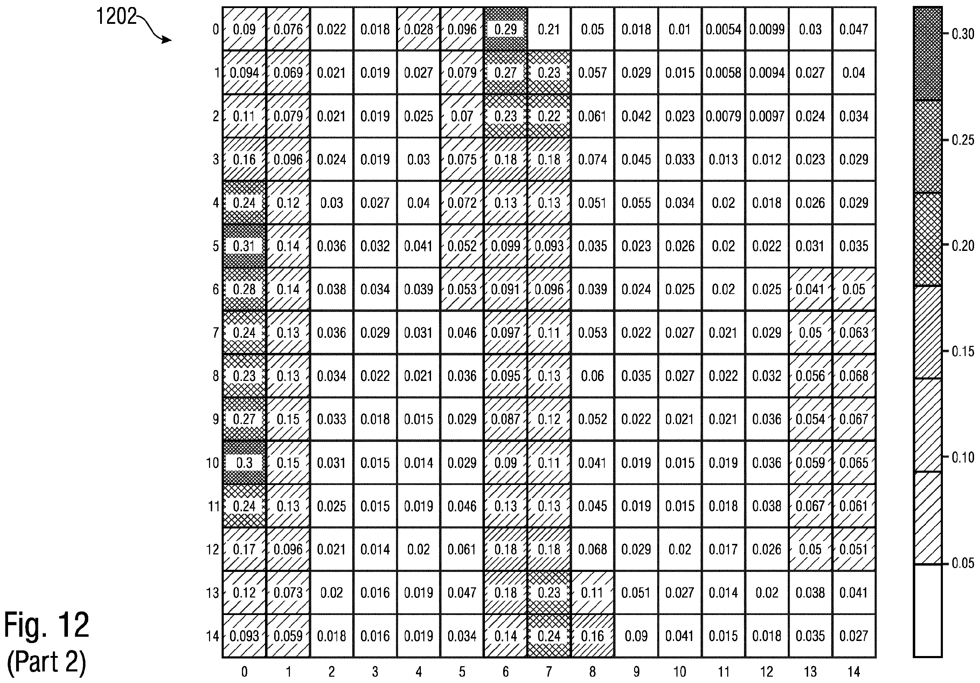

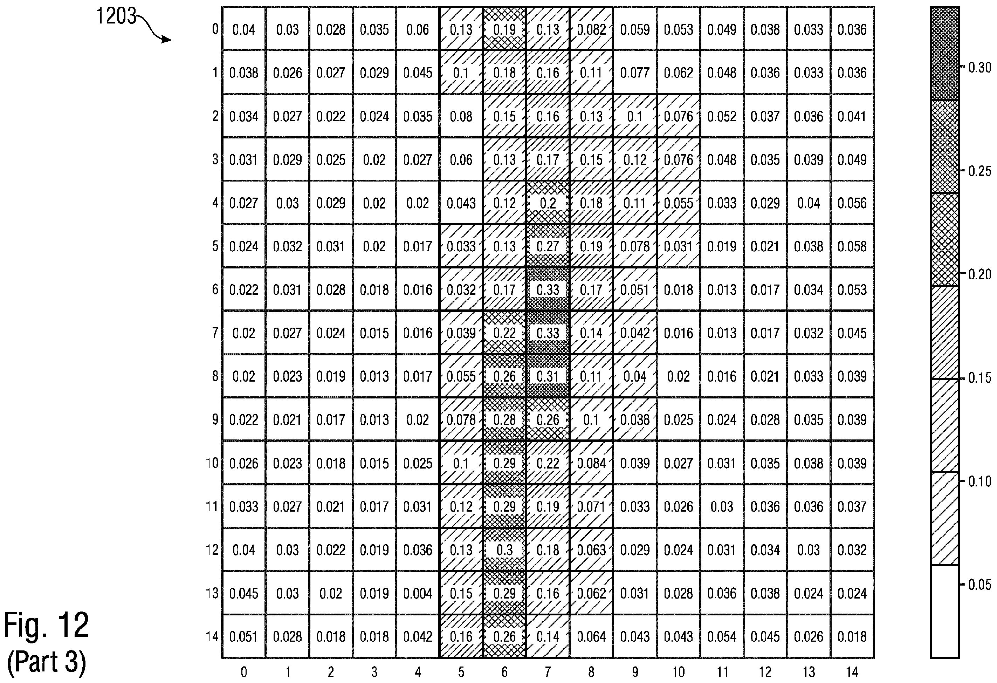

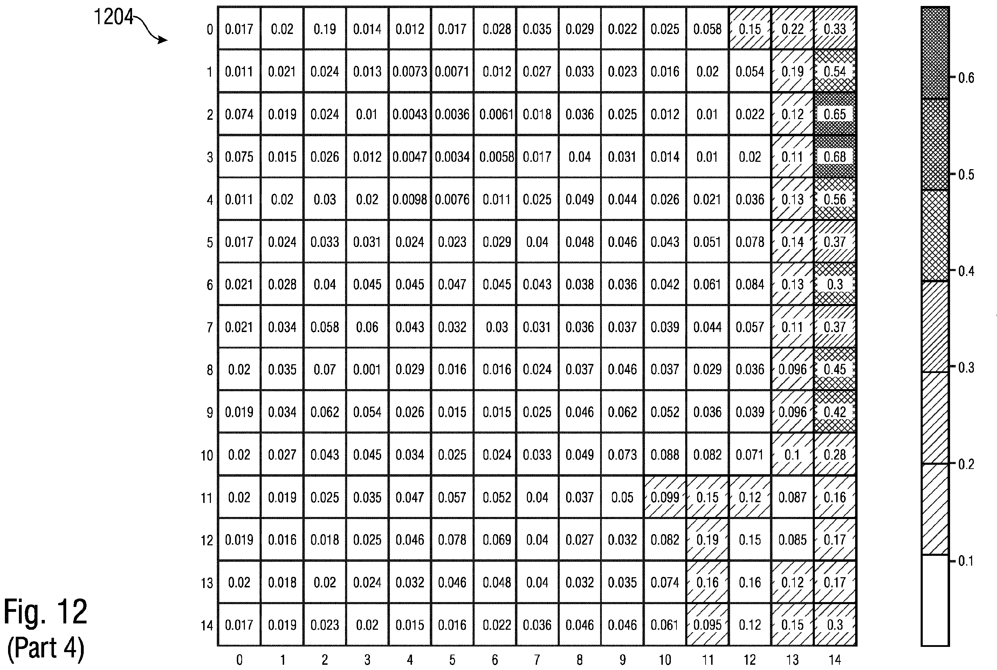

Fig. 12 illustrates attention maps 1201, 1202, 1203, and 1204 according to an example. Each of the attention maps indicates the portion of attention directed towards sensor signals with sequence length 15 broken out by one of four attention heads. In the attention maps, the abscissa denotes the time axis, 0 denotes the sensor signal furthest back in the past and 14 denotes the most current sensor signal. The ordinate denotes the feature dimension, while the attention are color-coded. In attention map 1201, the past sensor signals in the sequence are disproportionately targeted by the attention heads. For example, the four last sensor signals receive 60% of attention. In Head 2, map 1202, sensor timestep 0,1, 6 and 7 receive over 55% percent of the attention and in head 3, map 1203, sensor time steps 5, 6 and 7 receive on average over 50% of the attention. However, Head 4, map 1204, gives sensor timestep 13 and 14 over 60% of the attention. The attention heads that focuses on different sensor timesteps tend to cluster by sensor signals that are in proximity with respect to time. For example, head 1, 2 ,3 and 4 clusters the last, middle and latest timesteps by attention scores. Furthermore, the four attention maps show that sensor timesteps are important for gas prediction tasks and that generally more attention may be given to the middle and last sensor timesteps. This could mean that the timesteps from the past contain more information relevant for the gas prediction task than from the present or that present sensor signals' information is more intuitive to extract and does not need much attention to focus on and the timeseries sensor signals from the past need more attention in order to extract information from them.

Although some aspects have been described as features in the context of an apparatus it is clear that such a description may also be regarded as a description of corresponding features of a method. Although some aspects have been described as features in the context of a method, it is clear that such a description may also be regarded as a description of corresponding features concerning the functionality of an apparatus.

Some or all of the method steps or blocks, e.g. blocks 20, 30, 40, may be executed by (or using) a hardware apparatus, like for example, a microprocessor, a programmable computer or an electronic circuit. In some examples, one or more of the most important method steps may be executed by such an apparatus. In other words, modules 20 and 30, and neural network 40 may be executed by a processor, such as a signal processor, or microprocessor.

Depending on certain implementation requirements, examples of the disclosure can be implemented in hardware or in software or at least partially in hardware or at least partially in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some examples according to the disclosure comprise a data carrier having electronically readable control signals, which are capable of cooperating with a programmable computer system, such that one of the methods described herein is performed.

Generally, examples of the present disclosure can be implemented as a computer program product with a program code, the program code being operative for performing one of the methods when the computer program product runs on a computer. The program code may for example be stored on a machine readable carrier.

Other examples comprise the computer program for performing one of the methods described herein, stored on a machine readable carrier.

In other words, an example of the inventive method is, therefore, a computer program having a program code for performing one of the methods described herein, when the computer program runs on a computer.

A further example of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitory.

A further example of the inventive method is, therefore, a data stream or a sequence of signals representing the computer program for performing one of the methods described herein. The data stream or the sequence of signals may for example be configured to be transferred via a data communication connection, for example via the Internet.

A further example comprises a processing means, for example a computer, or a programmable logic device, configured to or adapted to perform one of the methods described herein.

A further example comprises a computer having installed thereon the computer program for performing one of the methods described herein.

A further example according to the disclosure comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some examples, a programmable logic device (for example a field programmable gate array) may be used to perform some or all of the functionalities of the methods described herein. In some examples, a field programmable gate array may cooperate with a microprocessor in order to perform one of the methods described herein. Generally, the methods are preferably performed by any hardware apparatus.

The apparatus described herein may be implemented using a hardware apparatus, or using a computer, or using a combination of a hardware apparatus and a computer.

The methods described herein may be performed using a hardware apparatus, or using a computer, or using a combination of a hardware apparatus and a computer.

In the foregoing Detailed Description, it can be seen that various features are grouped together in examples for the purpose of streamlining the disclosure. This method of disclosure is not to be interpreted as reflecting an intention that the claimed examples require more features than are expressly recited in each claim. Rather, as the following claims reflect, subject matter may lie in less than all features of a single disclosed example.

The above described examples are merely illustrative for the principles of the present disclosure. It is understood that modifications and variations of the arrangements and the details described herein will be apparent to others skilled in the art. It is the intent, therefore, to be limited only by the scope of the pending patent claims and not by the specific details presented by way of description and explanation of the examples herein.

References

[1] Vaswani, Ashish, et al. "Attention is all you need." Advances in neural information processing systems 30 (2017).

**Claims**

1. Gas sensing device (2) for sensing a target gas in a gas mixture, the gas sensing device comprising:

   a measurement module (10) configured for obtaining a measurement signal (12), the measurement signal being responsive to a concentration of the target gas in the gas mixture;
   a processing module (20) configured for

   determining (30), for each of a sequence (16) of samples (14) of the measurement signal (12), a set (32) of features, the features representing respective characteristics of the measurement signal;
   using a neural network (40) for determining an estimation (82) of the concentration of the target gas based on the sets (32) of features determined for the samples of the sequence (16);
   wherein the neural network (40) comprises an attention layer (43) to weight respective contributions of the samples to the estimation (82), wherein the attention layer (43) is configured for

   receiving, as input features (431) of the attention layer (43), for each of the samples (14), an associated set (37) of input features;
   determining (433), for each permutation of two of the samples, a respective weight based on the sets $(37_1, 37_2)$ of input features associated with the two samples of the respective permutation; and
   determining (439) a plurality of output features (432) of the attention layer (43) by using the weights for weighting contributions of the input features associated with the samples to the output features; and

   wherein the processing module is configured for determining the estimation (82) based on the output features of the attention layer (43).

2. Gas sensing device according to claim 1, wherein the attention layer (43) is configured for determining (433) weights for weighting the contribution of one of the samples to the estimation (82) based on the sets (32) of features determined for the samples of the sequence.

3. Gas sensing device according to claim 1 or 2, wherein the neural network (40) is configured for processing the sets (32) of features of the samples (14) of the sequence (16) in parallel.

4. Gas sensing device according to any of the preceding claims , wherein the attention layer (43) is configured for

   determining (435), for each of the samples, a first vector by applying a first trained weight matrix to the set of input features associated with the respective sample;
   determining (436), for each of the samples, a second vector by applying a second trained weight matrix to the set of input features associated with the respective sample; and
   determining (433) the respective weight for the respective permutation of two of the samples by forming a product of the first vector associated with one of the two samples and the second vector associated with the other one of the two samples.

5. Gas sensing device according to any of the preceding claims, wherein the attention layer (43) is configured for

   determining (437), for each of the samples, a third vector $(437_1, 437_T)$ by applying a third trained weight matrix to the set of input features associated with the respective sample;
   determining (439) the output features of the attention layer (43) by using the weights determined for the permutations for weighting the third vectors.

6. Gas sensing device according to claim 5,

wherein the weights (434) determined for the permutations form a weight matrix;
wherein the attention layer (43) is configured for concatenating the third vectors associated with the samples to form a value matrix (487); and
determining the output features (432) of the attention layer (43) by multiplying the weight matrix and the value matrix.

7. Gas sensing device according to any of the preceding claims, wherein the attention layer (43) is configured for normalizing the weights determined for the permutations and/or applies a softmax function to the weights.

8. Gas sensing device according to any of the preceding claims, wherein the attention layer (43) comprises a plurality of self-attention blocks (43'), wherein each of the self-attention blocks is configured for determining a respective plurality of output features (432') based on the input features (431) of the attention layer (43), and wherein the attention layer (43) is configured for determining the plurality of output features (432) of the attention layer (43) by using a fourth trained weight matrix for weighting contributions of the output features of the self-attention blocks to the output features of the attention layer (43).

9. Gas sensing device according to claim 8, wherein each of the self-attention blocks (43') is configured for

determining (433), for each permutation of two of the samples, a respective weight using the sets ($37_1$, $37_2$) of input features associated with the two samples of the respective permutation;
determining (435), for each of the samples, a first vector by applying a first trained weight matrix of the respective self-attention block to the set of input features associated with the respective sample;
determining (436), for each of the samples, a second vector by applying a second trained weight matrix of the respective self-attention block to the set of input features associated with the respective sample;
determining (433) the respective weight for the respective permutation of two of the samples by forming a product of the first vector associated with one of the two samples and the second vector associated with the other one of the two samples;
determining (437), for each of the samples, a third vector by applying a third trained weight matrix of the respective self-attention block to the set of input features associated with the respective sample; and
determining (439) the output features (432') of the self-attention block by using the weights determined for the permutations for weighting the third vectors.

10. Gas sensing device according to any of the preceding claims, wherein the attention layer (43) is configured for determining a plurality of output features of the attention layer (43) based on a plurality of input features of the attention layer (43), and wherein the neural network (40) is configured for
combining (44) the input features of the attention layer (43) with the plurality of output features of the attention layer (43) to obtain a plurality of combined features (442).

11. Gas sensing device according to claim 10, wherein the neural network (40) is configured for normalizing the combined features (442).

12. Gas sensing device according to any of the preceding claims, wherein the neural network (40) comprises a positional encoding layer (42) configured for

determining, for each of the samples, a set of positional coded features based on the set of features associated with the respective sample by coding, into the set of positional coded features, information about a position of the respective sample within the sequence of samples;
and using the positional coded features for input features of the attention layer (43).

13. Gas sensing device according to any of the preceding claims, wherein the neural network (40) comprises a feed forward layer (41) configured for applying a feed forward transformation to each of the sets of features associated with the samples, wherein input features of the attention layer (43) are based on output features of the feed forward layer.

14. Gas sensing device according to any of the preceding claims, wherein the measurement module (10) comprises one or more chemo-resistive gas sensing units to provide the measurement signal.

15. Method for sensing a target gas in a gas mixture, carried out by a device according to any of claims 1-14, the method comprising

obtaining (10) a measurement signal (12), the measurement signal being responsive to the concentration of the target gas in the gas mixture;

determining (30), for each of a sequence (16) of samples (14) of the measurement signal, a set (32) of features, the features representing respective characteristics of the measurement signal;

using an artificial neural network (40) for determining an estimation (82) of the concentration of the target gas based on the sets of features determined for the samples of the sequence;

wherein the artificial neural network (40) comprises an attention layer (43) to weight respective contributions of the samples to the estimation, wherein using the artificial neural network (40) comprises

receiving, as input features (431) of the attention layer (43), for each of the samples (14), an associated set (37) of input features;

determining (433), for each permutation of two of the samples, a respective weight based on the sets ($37_1$, $37_2$) of input features associated with the two samples of the respective permutation; and

determining (439) a plurality of output features (432) of the attention layer (43) by using the weights for weighting contributions of the input features associated with the samples to the output features; and

wherein the method comprises determining the estimation (82) based on the output features of the attention layer (43).

**Patentansprüche**

1. Gaserfassungsvorrichtung (2) zum Erfassen eines Zielgases in einem Gasgemisch, wobei die Gaserfassungsvorrichtung folgende Merkmale aufweist:

ein Messmodul (10), das dazu konfiguriert ist, ein Messsignal (12) zu erhalten, wobei das Messsignal auf eine Konzentration des Zielgases in dem Gasgemisch anspricht;

ein Verarbeitungsmodul (20), das zu Folgendem konfiguriert ist:

Bestimmen (30), für jede einer Sequenz (16) von Proben (14) des Messsignals (12), eines Satzes (32) von Merkmalen, wobei die Merkmale jeweilige Charakteristika des Messsignals darstellen;

Verwenden eines neuronalen Netzwerks (40) zum Bestimmen einer Schätzung (82) der Konzentration des Zielgases basierend auf den Sätzen (32) von Merkmalen, die für die Proben der Sequenz (16) bestimmt werden;

wobei das neuronale Netzwerk (40) eine Aufmerksamkeitsschicht (43) aufweist, um jeweilige Beiträge der Proben zu der Schätzung (82) zu gewichten, wobei die Aufmerksamkeitsschicht (43) zu Folgendem konfiguriert ist:

Empfangen, als Eingabemerkmale (431) der Aufmerksamkeitsschicht (43), für jede der Proben (14), eines zugeordneten Satzes (37) von Eingabemerkmalen;

Bestimmen (433), für jede Permutation von zwei der Proben, einer jeweiligen Gewichtung basierend auf den Sätzen ($37_1$, $37_2$) von Eingabemerkmalen, die den zwei Proben der jeweiligen Permutation zugeordnet sind; und

Bestimmen (439) einer Mehrzahl von Ausgabemerkmalen (432) der Aufmerksamkeitsschicht (43) durch Verwenden der Gewichtungen zum Gewichten von Beiträgen der Eingabemerkmale, die den Proben zugeordnet sind, zu den Ausgabemerkmalen; und

wobei das Verarbeitungsmodul dazu konfiguriert ist, die Schätzung (82) basierend auf den Ausgabemerkmalen der Aufmerksamkeitsschicht (43) zu bestimmen.

2. Gaserfassungsvorrichtung gemäß Anspruch 1, wobei die Aufmerksamkeitsschicht (43) dazu konfiguriert ist, Gewichtungen zum Gewichten des Beitrags einer der Proben zu der Schätzung (82) basierend auf den Sätzen (32) von Merkmalen, die für die Proben der Sequenz bestimmt werden, zu bestimmen (433).

3. Gaserfassungsvorrichtung gemäß Anspruch 1 oder 2, wobei das neuronale Netzwerk (40) dazu konfiguriert ist, die Sätze (32) von Merkmalen der Proben (14) der Sequenz (16) parallel zu verarbeiten.

4. Gaserfassungsvorrichtung gemäß einem der vorhergehenden Ansprüche, wobei die Aufmerksamkeitsschicht (43) zu Folgendem konfiguriert ist:

Bestimmen (435), für jede der Proben, eines ersten Vektors durch Anwenden einer ersten trainierten Gewichtungsmatrix auf den Satz von Eingabemerkmalen, die der jeweiligen Probe zugeordnet sind;
Bestimmen (436), für jede der Proben, eines zweiten Vektors durch Anwenden einer zweiten trainierten Gewichtungsmatrix auf den Satz von Eingabemerkmalen, die der jeweiligen Probe zugeordnet sind; und
Bestimmen (433) der jeweiligen Gewichtung für die jeweilige Permutation von zwei der Proben durch Bilden eines Produkts des ersten Vektors, der einer der zwei Proben zugeordnet ist, und des zweiten Vektors, der der anderen der zwei Proben zugeordnet ist.

5. Gaserfassungsvorrichtung gemäß einem der vorhergehenden Ansprüche, wobei die Aufmerksamkeitsschicht (43) zu Folgendem konfiguriert ist:

Bestimmen (437), für jede der Proben, eines dritten Vektors ($437_1$, $437_T$) durch Anwenden einer dritten trainierten Gewichtungsmatrix auf den Satz von Eingabemerkmalen, die der jeweiligen Probe zugeordnet sind;
Bestimmen (439) der Ausgabemerkmale der Aufmerksamkeitsschicht (43) durch Verwenden der Gewichtungen, die für die Permutationen zum Gewichten der dritten Vektoren bestimmt wurden.

6. Gaserfassungsvorrichtung gemäß Anspruch 5,

wobei die Gewichtungen (434), die für die Permutationen bestimmt wurden, eine Gewichtungsmatrix bilden;
wobei die Aufmerksamkeitsschicht (43) zum Verketten der dritten Vektoren, die den Proben zugeordnet sind, dazu konfiguriert ist, eine Wertematrix (487) zu bilden; und
Bestimmen der Ausgabemerkmale (432) der Aufmerksamkeitsschicht (43) durch Multiplizieren der Gewichtungsmatrix und der Wertematrix.

7. Gaserfassungsvorrichtung gemäß einem der vorhergehenden Ansprüche, wobei die Aufmerksamkeitsschicht (43) dazu konfiguriert ist, die Gewichtungen, die für die Permutationen bestimmt wurden, zu normalisieren und/oder eine Softmax-Funktion auf die Gewichtungen anzuwenden.

8. Gaserfassungsvorrichtung gemäß einem der vorhergehenden Ansprüche, wobei die Aufmerksamkeitsschicht (43) eine Mehrzahl von Selbstaufmerksamkeitsblöcken (43') aufweist, wobei jeder der Selbstaufmerksamkeitsblöcke dazu konfiguriert ist, eine jeweilige Mehrzahl von Ausgabemerkmalen (432') basierend auf den Eingabemerkmalen (431) der Aufmerksamkeitsschicht (43) zu bestimmen, und wobei die Aufmerksamkeitsschicht (43) dazu konfiguriert ist, die Mehrzahl von Ausgabemerkmalen (432) der Aufmerksamkeitsschicht (43) durch Verwenden einer vierten trainierten Gewichtungsmatrix zum Gewichten von Beiträgen der Ausgabemerkmale der Selbstaufmerksamkeitsblöcke zu den Ausgabemerkmalen der Aufmerksamkeitsschicht (43) zu bestimmen.

9. Gaserfassungsvorrichtung gemäß Anspruch 8, wobei jeder der Selbstaufmerksamkeitsblöcke (43') zu Folgendem konfiguriert ist:

Bestimmen (433), für jede Permutation von zwei der Proben, einer jeweiligen Gewichtung unter Verwendung der Sätze ($37_1$, $37_2$) von Eingabemerkmalen, die den zwei Proben der jeweiligen Permutation zugeordnet sind;
Bestimmen (435), für jede der Proben, eines ersten Vektors durch Anwenden einer ersten trainierten Gewichtungsmatrix des jeweiligen Selbstaufmerksamkeitsblocks auf den Satz von Eingabemerkmalen, die der jeweiligen Probe zugeordnet sind;
Bestimmen (436), für jede der Proben, eines zweiten Vektors durch Anwenden einer zweiten trainierten Gewichtungsmatrix des jeweiligen Selbstaufmerksamkeitsblocks auf den Satz von Eingabemerkmalen, die der jeweiligen Probe zugeordnet sind;
Bestimmen (433) der jeweiligen Gewichtung für die jeweilige Permutation von zwei der Proben durch Bilden eines Produkts des ersten Vektors, der einer der zwei Proben zugeordnet ist, und des zweiten Vektors, der der anderen der zwei Proben zugeordnet ist;
Bestimmen (437), für jede der Proben, eines dritten Vektors durch Anwenden einer dritten trainierten Gewichtungsmatrix des jeweiligen Selbstaufmerksamkeitsblocks auf den Satz von Eingabemerkmalen, die der jeweiligen Probe zugeordnet sind; und

Bestimmen (439) der Ausgabemerkmale (432') des Selbstaufmerksamkeitsblocks durch Verwenden der Gewichtungen, die für die Permutationen zum Gewichten der dritten Vektoren bestimmt wurden.

**10.** Gaserfassungsvorrichtung gemäß einem der vorhergehenden Ansprüche, wobei die Aufmerksamkeitsschicht (43) dazu konfiguriert ist, eine Mehrzahl von Ausgabemerkmalen der Aufmerksamkeitsschicht (43) basierend auf einer Mehrzahl von Eingabemerkmalen der Aufmerksamkeitsschicht (43) zu bestimmen, und wobei das neuronale Netzwerk (40) zu Folgendem konfiguriert ist:
Kombinieren (44) der Eingabemerkmale der Aufmerksamkeitsschicht (43) mit der Mehrzahl von Ausgabemerkmalen der Aufmerksamkeitsschicht (43), um eine Mehrzahl von kombinierten Merkmalen (442) zu erhalten.

**11.** Gaserfassungsvorrichtung gemäß Anspruch 10, wobei das neuronale Netzwerk (40) dazu konfiguriert ist, die kombinierten Merkmale (442) zu normalisieren.

**12.** Gaserfassungsvorrichtung gemäß einem der vorhergehenden Ansprüche, wobei das neuronale Netzwerk (40) eine Positionscodierungsschicht (42) aufweist, die zu Folgendem konfiguriert ist:

Bestimmen, für jede der Proben, eines Satzes von positionscodierten Merkmalen basierend auf dem Satz von Merkmalen, die der jeweiligen Probe zugeordnet sind, durch Codieren, in den Satz von positionscodierten Merkmalen, von Informationen über eine Position der jeweiligen Probe innerhalb der Sequenz von Proben; und Verwenden der positionscodierten Merkmale für Eingabemerkmale der Aufmerksamkeitsschicht (43).

**13.** Gaserfassungsvorrichtung gemäß einem der vorhergehenden Ansprüche, wobei das neuronale Netzwerk (40) eine Vorwärtskopplungsschicht (41) aufweist, die dazu konfiguriert ist, eine Vorwärtskopplungstransformation auf jeden der Sätze von Merkmalen, die den Proben zugeordnet sind, anzuwenden, wobei Eingabemerkmale der Aufmerksamkeitsschicht (43) auf Ausgabemerkmalen der Vorwärtskopplungsschicht basieren.

**14.** Gaserfassungsvorrichtung gemäß einem der vorhergehenden Ansprüche, wobei das Messmodul (10) eine oder mehrere chemoresistive Gaserfassungseinheiten aufweist, um das Messsignal bereitzustellen.

**15.** Verfahren zum Erfassen eines Zielgases in einem Gasgemisch, das durch eine Vorrichtung gemäß einem der Ansprüche 1 - 14 ausgeführt wird, wobei das Verfahren folgende Schritte aufweist:

Erhalten (10) eines Messsignals (12), wobei das Messsignal auf die Konzentration des Zielgases in dem Gasgemisch anspricht;
Bestimmen (30), für jede einer Sequenz (16) von Proben (14) des Messsignals, eines Satzes (32) von Merkmalen, wobei die Merkmale jeweilige Charakteristika des Messsignals darstellen;
Verwenden eines künstlichen neuronalen Netzwerks (40) zum Bestimmen einer Schätzung (82) der Konzentration des Zielgases basierend auf den Sätzen von Merkmalen, die für die Proben der Sequenz bestimmt werden;
wobei das künstliche neuronale Netzwerk (40) eine Aufmerksamkeitsschicht (43) aufweist, um jeweilige Beiträge der Proben zu der Schätzung zu gewichten, wobei das Verwenden des künstlichen neuronalen Netzwerks (40) folgende Schritte aufweist:

Empfangen, als Eingabemerkmale (431) der Aufmerksamkeitsschicht (43), für jede der Proben (14), eines zugeordneten Satzes (37) von Eingabemerkmalen;
Bestimmen (433), für jede Permutation von zwei der Proben, einer jeweiligen Gewichtung basierend auf den Sätzen ($37_1$, $37_2$) von Eingabemerkmalen, die den zwei Proben der jeweiligen Permutation zugeordnet sind; und
Bestimmen (439) einer Mehrzahl von Ausgabemerkmalen (432) der Aufmerksamkeitsschicht (43) durch Verwenden der Gewichtungen zum Gewichten von Beiträgen der Eingabemerkmale, die den Proben zugeordnet sind, zu den Ausgabemerkmalen; und

wobei das Verfahren das Bestimmen der Schätzung (82) basierend auf den Ausgabemerkmalen der Aufmerksamkeitsschicht (43) aufweist.

**Revendications**

**1.** Dispositif (2) de détection de gaz pour détecter un gaz cible dans un mélange de gaz, le dispositif de détection de gaz comprenant :

un module (10) de mesure configuré pour obtenir un signal (12) de mesure, le signal de mesure réagissant à une concentration du gaz cible dans le mélange de gaz ;
un module (20) de traitement configuré pour

déterminer (30), pour chacun d'une séquence (16) d'échantillons (14) du signal (12) de mesure, un ensemble (32) de particularités, les particularités représentant des caractéristiques respectives du signal de mesure ;
utiliser un réseau (40) neuronal pour déterminer une estimation (82) de la concentration du gaz cible sur la base des ensembles (32) de particularités déterminés pour les échantillons de la séquence (16) ;
dans lequel le réseau (40) neuronal comprend une couche (43) d'attention pour pondérer des contributions respectives des échantillons à l'estimation (82), dans lequel la couche (43) d'attention est configurée pour

recevoir, comme particularités (431) d'entrée de la couche (43) d'attention, pour chacun des échantillons (14), un ensemble (37) associé de particularités d'entrée ;
déterminer (433), pour chaque permutation de deux des échantillons, un poids respectif sur la base des ensembles ($37_1$, $37_2$) de particularités d'entrée associés aux deux échantillons de la permutation respective ; et
déterminer (439) une pluralité de particularités (432) de sortie de la couche (43) d'attention en utilisant les poids pour des contributions de pondération des particularités d'entrée associées aux échantillons aux particularités de sortie ; et

dans lequel le module de traitement est configuré pour déterminer l'estimation (82) sur la base des particularités de sortie de la couche (43) d'attention.

2. Dispositif de détection de gaz suivant la revendication 1, dans lequel la couche (43) d'attention est configurée pour déterminer (433) des poids pour pondérer la contribution de l'un des échantillons à l'estimation (82) sur la base des ensembles (32) de particularités déterminés pour les échantillons de la séquence.

3. Dispositif de détection de gaz suivant la revendication 1 ou 2, dans lequel le réseau (40) neuronal est configuré pour traiter les ensembles (32) de particularités des échantillons (14) de la séquence (16) en parallèle.

4. Dispositif de détection de gaz suivant l'une quelconque des revendications précédentes, dans lequel la couche (43) d'attention est configurée pour

déterminer (435), pour chacun des échantillons, un premier vecteur en appliquant une première matrice de poids entraînée à l'ensemble de particularités d'entrée associé à l'échantillon respectif ;
déterminer (436), pour chacun des échantillons, un deuxième vecteur en appliquant une deuxième matrice de poids entraînée à l'ensemble de particularités d'entrée associé à l'échantillon respectif ; et
déterminer (433) le poids respectif pour la permutation respective de deux des échantillons en formant un produit du premier vecteur associé à l'un des deux échantillons par le deuxième vecteur associé à l'autre des deux échantillons.

5. Dispositif de détection de gaz suivant l'une quelconque des revendications précédentes, dans lequel la couche (43) d'attention est configurée pour

déterminer (437), pour chacun des échantillons, un troisième vecteur ($437_1$, $437_T$) en appliquant une troisième matrice de poids entraînée à l'ensemble de particularités d'entrée associé à l'échantillon respectif ;
déterminer (439) les particularités de sortie de la couche (43) d'attention en utilisant les poids déterminés pour les permutations pour pondérer les troisièmes vecteurs.

6. Dispositif de détection de gaz suivant la revendication 5,

dans lequel les poids (434) déterminés pour les permutations forment une matrice de poids ;
dans lequel la couche (43) d'attention est configurée pour concaténer les troisièmes vecteurs associés aux échantillons pour former une matrice (487) de valeurs ; et
déterminer les particularités (432) de sortie de la couche (43) d'attention en multipliant la matrice de poids par la matrice de valeur.

7. Dispositif de détection de gaz suivant l'une quelconque des revendications précédentes, dans lequel la couche (43) d'attention est configurée pour normer les poids déterminés pour les permutations et/ou pour appliquer une fonction softmax aux poids.

8. Dispositif de détection de gaz suivant l'une quelconque des revendications précédentes, dans lequel la couche (43) d'attention comprend une pluralité de blocs (43') d'auto-attention, dans lequel chacun des blocs d'auto-attention est configuré pour déterminer une pluralité respective de particularités (432') de sortie sur la base des particularités (431) d'entrée de la couche (43) d'attention, et dans lequel la couche (43) d'attention est configurée pour déterminer la pluralité de particularités (432) de sortie de la couche (43) d'attention en utilisant une quatrième matrice de poids entraînée pour des contributions de pondération des particularités de sortie des blocs d'auto-attention aux particularités de sortie de la couche (43) d'attention.

9. Dispositif de détection de gaz suivant la revendication 8, dans lequel chacun des blocs (43') d'auto-attention est configuré pour

   déterminer (433), pour chaque permutation de deux des échantillons, un poids respectif en utilisant les ensembles ($37_1$, $37_2$) de particularités d'entrée associés aux deux échantillons de la permutation respective ;
   déterminer (435), pour chacun des échantillons, un premier vecteur en appliquant une première matrice de poids entraînée du bloc d'auto-attention respectif à l'ensemble de particularités d'entrée associé à l'échantillon respectif;
   déterminer (436), pour chacun des échantillons, un deuxième vecteur en appliquant une deuxième matrice de poids entraînée du bloc d'auto-attention respectif à l'ensemble de particularités d'entrée associé à l'échantillon respectif
   déterminer (433) le poids respectif pour la permutation respective de deux des échantillons en formant un produit du premier vecteur associé à l'un des deux échantillons par le deuxième vecteur associé à l'autre des deux échantillons;
   déterminer (437), pour chacun des échantillons, un troisième vecteur en appliquant une troisième matrice de poids entraînée du bloc d'auto-attention respectif à l'ensemble de particularités d'entrée associé à l'échantillon respectif; et
   déterminer (439) les particularités (432') de sortie du bloc d'auto-attention en utilisant les poids déterminés pour les permutations pour pondérer les troisièmes vecteurs.

10. Dispositif de détection de gaz suivant l'une quelconque des revendications précédentes, dans lequel la couche (43) d'attention est configurée pour déterminer une pluralité de particularités de sortie de la couche (43) d'attention sur la base d'une pluralité de particularités d'entrée de la couche (43) d'attention, et dans lequel le réseau (40) neuronal est configuré pour
combiner (44) les particularités d'entrée de la couche (43) d'attention à la pluralité de particularités de sortie de la couche (43) d'attention afin d'obtenir une pluralité de particularités (442) combinées.

11. Dispositif de détection de gaz suivant la revendication 10, dans lequel le réseau (40) neuronal est configuré pour normer les particularités (442) combinées.

12. Dispositif de détection de gaz suivant l'une des revendications précédentes, dans lequel le réseau (40) neuronal comprend une couche (42) de codage positionnelle configurée pour

   déterminer, pour chacun des échantillons, un ensemble de particularités codé positionnel sur la base de l'ensemble de particularités associé à l'échantillon respectif en codant, dans l'ensemble de particularités codé positionnel, de l'information sur une position de l'échantillon respectif dans la séquence d'échantillons ;
   et utiliser les particularités codées positionnelles pour des particularités d'entrée de la couche (43) d'attention.

13. Dispositif de détection de gaz suivant l'une des revendications précédentes, dans lequel le réseau (40) neuronal comprend une couche (41) en avant de charge configurée pour appliquer une transformation en avant de charge à chacun des ensembles de particularités associés aux échantillons, dans lequel des particularités d'entrée de la couche (43) d'attention reposent sur des particularités de sortie de la couche en avant de charge.

14. Dispositif de détection de gaz suivant l'une quelconque des revendications précédentes, dans lequel le module (10) de mesure comprend une ou plusieurs unités de détection de gaz chémio-résistantes pour donner le signal de mesure.

**15.** Procédé de détection d'un gaz cible dans un mélange de gaz effectué par un dispositif suivant l'une quelconque des revendications 1 à 14, le procédé comprenant :

obtenir (10) un signal (12) de mesure, le signal de mesure étant réactif à la concentration du gaz cible dans le mélange de gaz ;

déterminer (30), pour chacun d'une séquence (16) d'échantillons (14) du signal de mesure, un ensemble (32) de particularités, les particularités représentant des caractéristiques respectives du signal de mesure ;

utiliser un réseau (40) neuronal artificiel pour déterminer une estimation (82) de la concentration du gaz cible sur la base des ensembles de particularités déterminés pour les échantillons de la séquence ;

dans lequel un réseau (40) neuronal artificiel comprend une couche (43) d'attention pour pondérer des contributions respectives des échantillons à l'estimation, dans lequel utiliser le réseau (40) neuronal artificiel comprend

recevoir, comme particularités (431) d'entrée de la couche (43) d'attention, pour chacun des échantillons (14), un ensemble (37) associé de particularités d'entrée ;

déterminer (433), pour chaque permutation de deux des échantillons, un poids respectif sur la base des ensembles ($37_1$, $37_2$) de particularités d'entrée associés aux deux échantillons de la permutation respective ; et

déterminer (439) une pluralité de particularités (432) de sortie de la couche (43) d'attention en utilisant les pondérations pour pondérer des contributions des particularités d'entrée associées aux échantillons aux particularités de sortie ; et

dans lequel le procédé comprend déterminer l'estimation (82) sur la base des particularités de sortie de la couche (43) d'attention.

Fig. 1

Fig. 2

EP 4 368 987 B1

432

Row 1: $Z_{1\_1}$ $Z_{2\_1}$ $Z_{3\_1}$ $Z_{4\_1}$ $Z_{5\_1}$ $Z_{6\_1}$ $Z_{7\_1}$ $Z_{8\_1}$ $Z_{9\_1}$ $Z_{10\_1}$ $Z_{11\_1}$ $Z_{12\_1}$ $\cdots$ $Z_{T\_1}$

Row 2: $Z_{1\_2}$ $Z_{2\_2}$ $Z_{3\_2}$ $Z_{4\_2}$ $Z_{5\_2}$ $Z_{6\_2}$ $Z_{7\_2}$ $Z_{8\_2}$ $Z_{9\_2}$ $Z_{10\_2}$ $Z_{11\_2}$ $Z_{12\_2}$ $\cdots$ $Z_{T\_2}$

$\cdots$

Row M: $Z_{1\_M}$ $Z_{2\_M}$ $Z_{3\_M}$ $Z_{4\_M}$ $Z_{5\_M}$ $Z_{6\_M}$ $Z_{7\_M}$ $Z_{8\_M}$ $Z_{9\_M}$ $Z_{10\_M}$ $Z_{11\_M}$ $Z_{12\_M}$ $\cdots$ $Z_{T\_M}$

43

43'

439

$434_{12}$

$434_{11}$

434

$Q_1K_1$ $Q_{2\_K1}$ $Q_{3\_K1}$ $Q_{4\_K1}$ $Q_{5\_K1}$ $\cdots$ $Q_{T\_K1}$

$Q_1K_2$ $Q_{2\_K2}$ $Q_{3\_K2}$ $Q_{4\_K2}$ $Q_{5\_K2}$ $\cdots$ $Q_{T\_K2}$

$Q_1K_1$ $Q_{2\_K1}$ $Q_{3\_K1}$ $Q_{4\_K1}$ $Q_{5\_K1}$ $\cdots$ $Q_{T\_K1}$

$Q_1K_2$ $Q_{2\_K2}$ $Q_{3\_K2}$ $Q_{4\_K2}$ $Q_{5\_K2}$ $\cdots$ $Q_{T\_K2}$

$\cdots$

$Q_{1\_KM}$ $Q_{2\_KM}$ $Q_{3\_KM}$ $Q_{4\_KM}$ $Q_{5\_KM}$ $\cdots$ $Q_{T\_KM}$

self attention

Fig. 3 (Part 1)

Fig. 3 (Part 2)

43

$Z^1_{1\_1}$ $Z^1_{2\_1}$ $Z^1_{3\_1}$ $Z^1_{4\_1}$ $Z^1_{5\_1}$ • • • $Z^1_{T\_1}$ $Z^2_{1\_1}$ $Z^2_{2\_1}$ $Z^2_{3\_1}$ $Z^2_{4\_1}$ $Z^2_{5\_1}$ • • •

481

438

$Z^1_{1\_2}$ $Z^1_{2\_2}$ $Z^1_{3\_2}$ $Z^1_{4\_2}$ $Z^1_{5\_2}$ • • • $Z^1_{T\_2}$ $Z^2_{1\_2}$ $Z^2_{2\_2}$ $Z^2_{3\_2}$ $Z^2_{4\_2}$ $Z^2_{5\_2}$ • • •

• • •   • • •

483

$Z^1_{1\_M}$ $Z^1_{2\_M}$ $Z^1_{3\_M}$ $Z^1_{4\_M}$ $Z^1_{5\_M}$ • • • $Z^1_{T\_M}$ $Z^2_{1\_M}$ $Z^2_{2\_M}$ $Z^2_{3\_M}$ $Z^2_{4\_M}$ $Z^2_{5\_M}$ • • •

432'

$Z^1_{1\_1}$ $Z^1_{2\_1}$ $Z^1_{3\_1}$ $Z^1_{4\_1}$ $Z^1_{5\_1}$ • • • $Z^1_{T\_1}$    432'   $Z^2_{1\_1}$ $Z^2_{2\_1}$ $Z^2_{3\_1}$ $Z^2_{4\_1}$ $Z^2_{5\_1}$ • • • $Z^2_{T\_1}$

$Z^1_{1\_2}$ $Z^1_{2\_2}$ $Z^1_{3\_2}$ $Z^1_{4\_2}$ $Z^1_{5\_2}$ • • • $Z^1_{T\_2}$    $Z^2_{1\_2}$ $Z^2_{2\_2}$ $Z^2_{3\_2}$ $Z^2_{4\_2}$ $Z^2_{5\_2}$ • • • $Z^2_{T\_2}$

• • •   • • •

$Z^1_{1\_M}$ $Z^1_{2\_M}$ $Z^2_{3\_M}$ $Z^1_{4\_M}$ $Z^1_{5\_M}$ • • • $Z^1_{T\_M}$    $Z^2_{1\_M}$ $Z^2_{2\_M}$ $Z^2_{3\_M}$ $Z^2_{4\_M}$ $Z^2_{5\_M}$ • • • $Z^2_{T\_M}$

43'   self attention (1)   • • •   self attention (2)   43'

Fig. 4 (Part 1)

Fig. 4 (Part 2)

40

82

| NO2 and O3 concentration |

48 — | feed forward |

47 — | flatten |

492 — 49

46 — | ADD and normalize |

445

45 — | |

442

44 — | ADD and normalize |

432

43 — | multi-head attention |

444

431

42 — | positional encoding |

412

41 — | position wise feed forward |

32

| sensor feature timestep 1 | sensor feature timestep 2 | sensor feature timestep 3 | sensor feature timestep 4 | sensor feature timestep 5 | sensor feature timestep 6 | ... | sensor feature timestep T-1 | sensor feature timestep T |

32

**Fig. 5**

Fig. 6

Fig. 7

422

| $\Gamma_{1\_1}$ | $\Gamma_{2\_1}$ | $\Gamma_{3\_1}$ | $\Gamma_{4\_1}$ | $\Gamma_{5\_1}$ | $\Gamma_{6\_1}$ | $\Gamma_{7\_1}$ | $\Gamma_{8\_1}$ | $\Gamma_{9\_1}$ | $\Gamma_{10\_1}$ | $\Gamma_{11\_1}$ | $\Gamma_{12\_1}$ | $\cdots$ | $\Gamma_{T\_1}$ |
| $\Gamma_{1\_2}$ | $\Gamma_{2\_2}$ | $\Gamma_{3\_2}$ | $\Gamma_{4\_2}$ | $\Gamma_{5\_2}$ | $\Gamma_{6\_2}$ | $\Gamma_{7\_2}$ | $\Gamma_{8\_2}$ | $\Gamma_{9\_2}$ | $\Gamma_{10\_1}$ | $\Gamma_{11\_2}$ | $\Gamma_{12\_2}$ | $\cdots$ | $\Gamma_{T\_2}$ |

$\cdots$

| $\Gamma_{1\_M}$ | $\Gamma_{2\_M}$ | $\Gamma_{3\_M}$ | $\Gamma_{4\_M}$ | $\Gamma_{5\_M}$ | $\Gamma_{6\_M}$ | $\Gamma_{7\_M}$ | $\Gamma_{8\_M}$ | $\Gamma_{9\_M}$ | $\Gamma_{10\_M}$ | $\Gamma_{11\_M}$ | $\Gamma_{12\_M}$ | $\cdots$ | $\Gamma_{T\_M}$ |

42

**positional encoding**

| $I_{1\_1}$ | $I_{2\_1}$ | $I_{3\_1}$ | $I_{4\_1}$ | $I_{5\_1}$ | $I_{6\_1}$ | $I_{7\_1}$ | $I_{8\_1}$ | $I_{9\_1}$ | $I_{10\_1}$ | $I_{11\_1}$ | $I_{12\_1}$ | $\cdots$ | $I_{T\_1}$ |
| $I_{1\_2}$ | $I_{2\_2}$ | $I_{3\_2}$ | $I_{4\_2}$ | $I_{5\_2}$ | $I_{6\_2}$ | $I_{7\_2}$ | $I_{8\_2}$ | $I_{9\_2}$ | $I_{10\_1}$ | $I_{11\_2}$ | $I_{12\_2}$ | $\cdots$ | $I_{T\_2}$ |

$\cdots$

| $I_{1\_M}$ | $I_{2\_M}$ | $I_{3\_M}$ | $I_{4\_M}$ | $I_{5\_M}$ | $I_{6\_M}$ | $I_{7\_M}$ | $I_{8\_M}$ | $I_{9\_M}$ | $I_{10\_M}$ | $I_{11\_M}$ | $I_{12\_M}$ | $\cdots$ | $I_{T\_M}$ |

424

426

| $P_{1\_1}$ | $P_{2\_1}$ | $P_{3\_1}$ | $P_{4\_1}$ | $P_{5\_1}$ | $P_{6\_1}$ | $P_{7\_1}$ | $P_{8\_1}$ | $P_{9\_1}$ | $P_{10\_1}$ | $P_{11\_1}$ | $P_{12\_1}$ | $\cdots$ | $P_{T\_1}$ |
| $P_{1\_2}$ | $P_{2\_2}$ | $P_{3\_2}$ | $P_{4\_2}$ | $P_{5\_2}$ | $P_{6\_2}$ | $P_{7\_2}$ | $P_{8\_2}$ | $P_{9\_2}$ | $P_{10\_1}$ | $P_{11\_2}$ | $P_{12\_2}$ | $\cdots$ | $P_{T\_2}$ |

$\cdots$

| $P_{1\_M}$ | $P_{2\_M}$ | $P_{3\_M}$ | $P_{4\_M}$ | $P_{5\_M}$ | $P_{6\_M}$ | $P_{7\_M}$ | $P_{8\_M}$ | $P_{9\_M}$ | $P_{10\_M}$ | $P_{11\_M}$ | $P_{12\_M}$ | $\cdots$ | $P_{T\_M}$ |

$+$

| $I_{1\_1}$ | $I_{2\_1}$ | $I_{3\_1}$ | $I_{4\_1}$ | $I_{5\_1}$ | $I_{6\_1}$ | $I_{7\_1}$ | $I_{8\_1}$ | $I_{9\_1}$ | $I_{10\_1}$ | $I_{11\_1}$ | $I_{12\_1}$ | $\cdots$ | $I_{T\_1}$ |
| $I_{1\_2}$ | $I_{2\_2}$ | $I_{3\_2}$ | $I_{4\_2}$ | $I_{5\_2}$ | $I_{6\_2}$ | $I_{7\_2}$ | $I_{8\_2}$ | $I_{9\_2}$ | $I_{10\_1}$ | $I_{11\_2}$ | $I_{12\_2}$ | $\cdots$ | $I_{T\_2}$ |

$\cdots$

| $I_{1\_M}$ | $I_{2\_M}$ | $I_{3\_M}$ | $I_{4\_M}$ | $I_{5\_M}$ | $I_{6\_M}$ | $I_{7\_M}$ | $I_{8\_M}$ | $I_{9\_M}$ | $I_{10\_M}$ | $I_{11\_M}$ | $I_{12\_M}$ | $\cdots$ | $I_{T\_M}$ |

424

Fig. 8

| $J_{1\_1}$ | $J_{2\_1}$ | $J_{3\_1}$ | $J_{4\_1}$ | $J_{5\_1}$ | $J_{6\_1}$ | $J_{7\_1}$ | $J_{8\_1}$ | $J_{9\_1}$ | $J_{10\_1}$ |

• • •

| $J_{14\_M}$ | $J_{15\_M}$ |

48

flatten

| $J_{1\_1}$ | $J_{2\_1}$ | $J_{3\_1}$ | $J_{4\_1}$ | $J_{5\_1}$ | $J_{6\_1}$ | $J_{7\_1}$ | $J_{8\_1}$ | $J_{9\_1}$ | $J_{10\_1}$ | $J_{11\_1}$ | $J_{12\_1}$ | $J_{13\_1}$ | $J_{14\_1}$ | $J_{15\_1}$ |
| $J_{1\_2}$ | $J_{2\_2}$ | $J_{3\_2}$ | $J_{4\_2}$ | $J_{5\_2}$ | $J_{6\_2}$ | $J_{7\_2}$ | $J_{8\_2}$ | $J_{9\_2}$ | $J_{10\_2}$ | $J_{11\_2}$ | $J_{12\_2}$ | $J_{13\_2}$ | $J_{14\_2}$ | $J_{15\_2}$ |

• • •

| $J_{1\_M}$ | $J_{2\_M}$ | $J_{3\_M}$ | $J_{4\_M}$ | $J_{5\_M}$ | $J_{6\_M}$ | $J_{7\_M}$ | $J_{8\_M}$ | $J_{9\_M}$ | $J_{10\_M}$ | $J_{11\_M}$ | $J_{12\_M}$ | $J_{13\_M}$ | $J_{14\_M}$ | $J_{15\_M}$ |

## Fig. 9

Fig. 10

Fig. 11

Fig. 12
(Part 1)

1201

1202

| | 0 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 0 | 0.09 | 0.076 | 0.022 | 0.018 | 0.028 | 0.096 | 0.29 | 0.21 | 0.05 | 0.018 | 0.01 | 0.0054 | 0.0099 | 0.03 | 0.047 |
| 1 | 0.094 | 0.069 | 0.021 | 0.019 | 0.027 | 0.079 | 0.27 | 0.23 | 0.057 | 0.029 | 0.015 | 0.0058 | 0.0094 | 0.027 | 0.04 |
| 2 | 0.11 | 0.079 | 0.021 | 0.019 | 0.025 | 0.07 | 0.23 | 0.22 | 0.061 | 0.042 | 0.023 | 0.0079 | 0.0097 | 0.024 | 0.034 |
| 3 | 0.16 | 0.096 | 0.024 | 0.019 | 0.03 | 0.075 | 0.18 | 0.18 | 0.074 | 0.045 | 0.033 | 0.013 | 0.012 | 0.023 | 0.029 |
| 4 | 0.24 | 0.12 | 0.03 | 0.027 | 0.04 | 0.072 | 0.13 | 0.13 | 0.051 | 0.055 | 0.034 | 0.02 | 0.018 | 0.026 | 0.029 |
| 5 | 0.31 | 0.14 | 0.036 | 0.032 | 0.041 | 0.052 | 0.099 | 0.093 | 0.035 | 0.023 | 0.026 | 0.02 | 0.022 | 0.031 | 0.035 |
| 6 | 0.28 | 0.14 | 0.038 | 0.034 | 0.039 | 0.053 | 0.091 | 0.096 | 0.039 | 0.024 | 0.025 | 0.02 | 0.025 | 0.041 | 0.05 |
| 7 | 0.24 | 0.13 | 0.036 | 0.029 | 0.031 | 0.046 | 0.097 | 0.11 | 0.053 | 0.022 | 0.027 | 0.021 | 0.029 | 0.05 | 0.063 |
| 8 | 0.23 | 0.13 | 0.034 | 0.022 | 0.021 | 0.036 | 0.095 | 0.13 | 0.06 | 0.035 | 0.027 | 0.022 | 0.032 | 0.056 | 0.068 |
| 9 | 0.27 | 0.15 | 0.033 | 0.018 | 0.015 | 0.029 | 0.087 | 0.12 | 0.052 | 0.022 | 0.021 | 0.021 | 0.036 | 0.054 | 0.067 |
| 10 | 0.3 | 0.15 | 0.031 | 0.015 | 0.014 | 0.029 | 0.09 | 0.11 | 0.041 | 0.019 | 0.015 | 0.019 | 0.036 | 0.059 | 0.065 |
| 11 | 0.24 | 0.13 | 0.025 | 0.015 | 0.019 | 0.046 | 0.13 | 0.13 | 0.045 | 0.019 | 0.015 | 0.018 | 0.038 | 0.067 | 0.061 |
| 12 | 0.17 | 0.096 | 0.021 | 0.014 | 0.02 | 0.061 | 0.18 | 0.18 | 0.068 | 0.029 | 0.02 | 0.017 | 0.026 | 0.05 | 0.051 |
| 13 | 0.12 | 0.073 | 0.02 | 0.016 | 0.019 | 0.047 | 0.18 | 0.23 | 0.11 | 0.051 | 0.027 | 0.014 | 0.02 | 0.038 | 0.041 |
| 14 | 0.093 | 0.059 | 0.018 | 0.016 | 0.019 | 0.034 | 0.14 | 0.24 | 0.16 | 0.09 | 0.041 | 0.015 | 0.018 | 0.035 | 0.027 |

Legend: 0.30 — 0.25 — 0.20 — 0.15 — 0.10 — 0.05

## Fig. 12
(Part 2)

1203

Legend scale: 0.30 | 0.25 | 0.20 | 0.15 | 0.10 | 0.05

| | 0 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 0 | 0.04 | 0.03 | 0.028 | 0.035 | 0.06 | 0.13 | 0.19 | 0.13 | 0.082 | 0.059 | 0.053 | 0.049 | 0.038 | 0.033 | 0.036 |
| 1 | 0.038 | 0.026 | 0.027 | 0.029 | 0.045 | 0.1 | 0.18 | 0.16 | 0.11 | 0.077 | 0.062 | 0.048 | 0.036 | 0.033 | 0.036 |
| 2 | 0.034 | 0.027 | 0.022 | 0.024 | 0.035 | 0.08 | 0.15 | 0.16 | 0.13 | 0.1 | 0.076 | 0.052 | 0.037 | 0.036 | 0.041 |
| 3 | 0.031 | 0.029 | 0.025 | 0.02 | 0.027 | 0.06 | 0.13 | 0.17 | 0.15 | 0.12 | 0.076 | 0.048 | 0.035 | 0.039 | 0.049 |
| 4 | 0.027 | 0.03 | 0.029 | 0.02 | 0.02 | 0.043 | 0.12 | 0.2 | 0.18 | 0.11 | 0.055 | 0.033 | 0.029 | 0.04 | 0.056 |
| 5 | 0.024 | 0.032 | 0.031 | 0.02 | 0.017 | 0.033 | 0.13 | 0.27 | 0.19 | 0.078 | 0.031 | 0.019 | 0.021 | 0.038 | 0.058 |
| 6 | 0.022 | 0.031 | 0.028 | 0.018 | 0.016 | 0.032 | 0.17 | 0.33 | 0.17 | 0.051 | 0.018 | 0.013 | 0.017 | 0.034 | 0.053 |
| 7 | 0.02 | 0.027 | 0.024 | 0.015 | 0.016 | 0.039 | 0.22 | 0.33 | 0.14 | 0.042 | 0.016 | 0.013 | 0.017 | 0.032 | 0.045 |
| 8 | 0.02 | 0.023 | 0.019 | 0.013 | 0.017 | 0.055 | 0.26 | 0.31 | 0.11 | 0.04 | 0.02 | 0.016 | 0.021 | 0.033 | 0.039 |
| 9 | 0.022 | 0.021 | 0.017 | 0.013 | 0.02 | 0.078 | 0.28 | 0.26 | 0.1 | 0.038 | 0.025 | 0.024 | 0.028 | 0.035 | 0.039 |
| 10 | 0.026 | 0.023 | 0.018 | 0.015 | 0.025 | 0.1 | 0.29 | 0.22 | 0.084 | 0.039 | 0.027 | 0.031 | 0.035 | 0.038 | 0.039 |
| 11 | 0.033 | 0.027 | 0.021 | 0.017 | 0.031 | 0.12 | 0.29 | 0.19 | 0.071 | 0.033 | 0.026 | 0.03 | 0.036 | 0.036 | 0.037 |
| 12 | 0.04 | 0.03 | 0.022 | 0.019 | 0.036 | 0.13 | 0.3 | 0.18 | 0.063 | 0.029 | 0.024 | 0.031 | 0.034 | 0.03 | 0.032 |
| 13 | 0.045 | 0.03 | 0.02 | 0.019 | 0.004 | 0.15 | 0.29 | 0.16 | 0.062 | 0.031 | 0.028 | 0.036 | 0.038 | 0.024 | 0.024 |
| 14 | 0.051 | 0.028 | 0.018 | 0.018 | 0.042 | 0.16 | 0.26 | 0.14 | 0.064 | 0.043 | 0.043 | 0.054 | 0.045 | 0.026 | 0.018 |

Fig. 12
(Part 3)

EP 4 368 987 B1

1204

| | 0 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 0 | 0.017 | 0.02 | 0.19 | 0.014 | 0.012 | 0.017 | 0.028 | 0.035 | 0.029 | 0.022 | 0.025 | 0.058 | 0.15 | 0.22 | 0.33 |
| 1 | 0.011 | 0.021 | 0.024 | 0.013 | 0.0073 | 0.0071 | 0.012 | 0.027 | 0.033 | 0.023 | 0.016 | 0.02 | 0.054 | 0.19 | 0.54 |
| 2 | 0.074 | 0.019 | 0.024 | 0.01 | 0.0043 | 0.0036 | 0.0061 | 0.018 | 0.036 | 0.025 | 0.012 | 0.01 | 0.022 | 0.12 | 0.65 |
| 3 | 0.075 | 0.015 | 0.026 | 0.012 | 0.0047 | 0.0034 | 0.0058 | 0.017 | 0.04 | 0.031 | 0.014 | 0.01 | 0.02 | 0.11 | 0.68 |
| 4 | 0.011 | 0.02 | 0.03 | 0.02 | 0.0098 | 0.0076 | 0.011 | 0.025 | 0.049 | 0.044 | 0.026 | 0.021 | 0.036 | 0.13 | 0.56 |
| 5 | 0.017 | 0.024 | 0.033 | 0.031 | 0.024 | 0.023 | 0.029 | 0.04 | 0.048 | 0.046 | 0.043 | 0.051 | 0.078 | 0.14 | 0.37 |
| 6 | 0.021 | 0.028 | 0.04 | 0.045 | 0.045 | 0.047 | 0.045 | 0.043 | 0.038 | 0.036 | 0.042 | 0.061 | 0.084 | 0.13 | 0.3 |
| 7 | 0.021 | 0.034 | 0.058 | 0.06 | 0.043 | 0.032 | 0.03 | 0.031 | 0.036 | 0.037 | 0.039 | 0.044 | 0.057 | 0.11 | 0.37 |
| 8 | 0.02 | 0.035 | 0.07 | 0.001 | 0.029 | 0.016 | 0.016 | 0.024 | 0.037 | 0.046 | 0.037 | 0.029 | 0.036 | 0.096 | 0.45 |
| 9 | 0.019 | 0.034 | 0.062 | 0.054 | 0.026 | 0.015 | 0.015 | 0.025 | 0.046 | 0.062 | 0.052 | 0.036 | 0.039 | 0.096 | 0.42 |
| 10 | 0.02 | 0.027 | 0.043 | 0.045 | 0.034 | 0.025 | 0.024 | 0.033 | 0.049 | 0.073 | 0.088 | 0.082 | 0.071 | 0.1 | 0.28 |
| 11 | 0.02 | 0.019 | 0.025 | 0.035 | 0.047 | 0.057 | 0.052 | 0.04 | 0.037 | 0.05 | 0.099 | 0.15 | 0.12 | 0.087 | 0.16 |
| 12 | 0.019 | 0.016 | 0.018 | 0.025 | 0.046 | 0.078 | 0.069 | 0.04 | 0.027 | 0.032 | 0.082 | 0.19 | 0.15 | 0.085 | 0.17 |
| 13 | 0.02 | 0.018 | 0.02 | 0.024 | 0.032 | 0.046 | 0.048 | 0.04 | 0.032 | 0.035 | 0.074 | 0.16 | 0.16 | 0.12 | 0.17 |
| 14 | 0.017 | 0.019 | 0.023 | 0.02 | 0.015 | 0.016 | 0.022 | 0.036 | 0.046 | 0.046 | 0.061 | 0.095 | 0.12 | 0.15 | 0.3 |

Fig. 12
(Part 4)

RNN algorithm

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PAN XIAOFANG et al.** A fast and robust mixture gases identification and concentration detection algorithm based on attention mechanism equipped recurrent neural network with double loss function. *SENSORS AND ACTUATORS B: CHEMICAL*, 24 April 2021, vol. 342

- **VASWANI ; ASHISH et al.** Attention is all you need. *Advances in neural information processing systems*, 2017, vol. 30